(19) Europäisches Patentamt

European Patent Office

Office européen des brevets

(11) **EP 0 726 944 B1**

(12) **EUROPEAN PATENT SPECIFICATION**

(45) Date of publication and mention
of the grant of the patent:
**18.12.2002 Bulletin 2002/51**

(51) Int Cl.⁷: **C07K 14/47**, C12N 15/12,
C12N 1/21

(21) Application number: **94921451.4**

(22) Date of filing: **01.07.1994**

(86) International application number:
**PCT/US94/07466**

(87) International publication number:
**WO 95/001428 (12.01.1995 Gazette 1995/03)**

(54) **GLYCOSYLATED AND NON-GLYCOSYLATED BACTERICIDAL/PERMEABILITY INCREASING PROTEINS, AND METHODS FOR PRODUCING SAME**

GLYCOSILIERTE UND NICHTGLYCOLISIERTE BAKTERIZIDE/DIE PERMEABILITÄT ERHOEHENDE PROTEINE UND METHODEN ZU IHRER HERSTELLUNG

PROTEINES GLYCOSYLEES ET NON GLYCOSYLEES, BACTERICIDES ET AUGMENTANT LA PERMEABILITE, ET PROCEDE DE PRODUCTION

(84) Designated Contracting States:
**AT BE CH DE DK ES FR GB GR IE IT LI NL PT SE**

(30) Priority: **02.07.1993 US 87484**

(43) Date of publication of application:
**21.08.1996 Bulletin 1996/34**

(73) Proprietor: **INCYTE PHARMACEUTICALS, INC.**
**Palo Alto , CA 94304 (US)**

(72) Inventors:
- **MARRA, Marian, N.**
**San Mateo, CA 94403 (US)**
- **SCOTT, Randal, W.**
**Mountain View, CA 94040 (US)**
- **LANE, John, C.**
**Menlo Park, CA 94025 (US)**
- **SNABLE, James, L.**
**Sunnyvale, CA 94086 (US)**

(74) Representative: **Richardson, Kate et al**
**Forrester & Boehmert,**
**Pettenkoferstrasse 20-22**
**80336 München (DE)**

(56) References cited:
**US-A- 5 171 739**

- **BIOTECHNOLOGY, vol. 7, November 1989, pages 1141-1149, XP000068927 SCHEIN, C.H.: "Production of soluble recombinant proteins in bacteria."**
- **JOURNAL OF CLINICAL INVESTIGATION, March 1983, pages 540-549, XP000611417 WEISS J. ET AL.: "Role of charge and hydrophobic interactions in the action of the Bactericidal/Permeability-increasing protein of Neutrophils on gram-negative bacteria."**
- **THE JOURNAL OF BIOLOGICAL CHEMISTRY, Vol. 264, No. 16, issued 05 June 1989, GRAY et al., "Cloning of the cDNA of a Human Neutrophil Protein. Structural and Functional Correlations", pages 9505-9509.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Background of the Invention

Gram-negative infections are a major cause of morbidity and mortality, especially in hospitalized and immunocompromised patients. [Duma, R.J., Am. J. of Med., 78 (Suppl. 6A): 154-164 (1985); and Kreger B.E., D.E. Craven and W.R. McCabe, Am. J. Med., 68: 344-355 (1980)]. Although available antibiotics are effective in containing gram-negative infections, they do nothing to neutralize the pathophysiological effects associated with lipopolysaccharide (LPS). LPS, also referred to herein as endotoxin, is a major component of the outer membrane of gram-negative bacteria and is released when the organisms are lysed. [Ahenep, J.L. and K.A. Morgan, J. Infect. Dis., 150 (3): 380-388 (1984)].

LPS released during antibiotic therapy is a potent stimulator of the inflammatory response. Many detrimental effects of LPS in vivo result from soluble mediators released by inflammatory cells. [Morrison D.C. and R.J. Ulevich, Am. J. Pathol., 93 (2): 527-617 (1978)). LPS induces the release of mediators by host inflammatory cells which may ultimately result in disseminated intravascular coagulation (DIC), adult respiratory distress syndrome (ARDS), renal failure and irreversible shock.

Monocytes and neutrophilic granulocytes play a key role in host defense against bacterial infections and also participate in the pathology of endotoxemia. These cells ingest and kill microorganisms intracellularly and also respond to LPS in vivo and in vitro by releasing soluble proteins with microbicidal, proteolytic, opsonic, pyrogenic, complement-activating and tissue-damaging effects. Tumor necrosis factor (TNF), a cytokine released by LPS-stimulated monocytes, mimics some of the toxic effects of LPS in vivo. Injecting animals with TNF causes fever, shock and alterations in glucose metabolism. TNF is also a potent stimulator of neutrophils. Other cytokines, such as IL-1, IL-6 and IL-8, are also released and may play a role in the inflammatory response to LPS.

Soluble LPS causes decreased neutrophil chemotaxis, increased adhesiveness, elevated hexose monophos-phate shunt activity and $O_2$ radical production, upregulation of surface receptors for complement, and release of granule proteins into the surrounding medium. [Morrison and Ulevich (1978)].

Both specific and azurophil compartments degranulate in response to LPS. [Bannatyne, R.M., N.M. Harnett, K.Y. Lee and W.D. Rigger, J. Infect. Dis., 156 (4):469-474 (1977)]. Azurophil proteins released in response to LPS may be both harmful and beneficial to the host. Neutrophil elastase causes degradation of protease inhibitors responsible for suppressing the coagulation cascade. This results in coagulopathies such as disseminated intravascular coagulation, a potentially lethal consequence of endotoxemia. Azurophil granules also contain bactericidal molecules such as myeloperoxidase and Bactericidal/Permeability Increasing Factor, hereinafter referred to as BPI Protein.

Rabbit BPI was first discovered in 1975. [Weiss, J., R.C. Franson, S. Becherdite, K. Schmeidler, and P. Elsbach, J. Clin. Invest., 55:33 (1975)]. BPI Protein was isolated from human neutrophils in 1978. [Weiss, J., P. Elsbach, I. Olson and H. Odeberg, J. Biol. Chem, 253 (8):2664-2672 (1978)].

In 1984 a 57 kD protein with similar properties was isolated from human neutrophils. [Shafer, W.M., C.E. Martin and J.K. Spitznagel, Infect. Immun., 45:29 (1984)]. This protein is identical to BPI Protein as determined by the 57 kDa protein's N-Terminal sequence, amino acid composition, molecular weight and source. Although, the authors were unable to reproduce the chromatographic isolation procedure used by Elsbach, et al. and Weiss, et al.

Human BPI Protein is a 57 kD protein which binds to the outer membrane of susceptible gram-negative bacteria. [Weiss, et al. (1978)]. The fact that BPI Protein is a Lipid A-binding protein is evidenced by: (1) rough strains of bacteria are more sensitive to both bactericidal and permeability increasing activities of BPI Protein [Weiss, J., M. Hutzler and L. Kao, Infect. Immun., 51:594 (1986)]; (2) mutations in Lipid A cause decreased binding and increase resistance to bactericidal activity of both polymyxin B and BPI Protein [Farley, M.M., W.M. Shafer and J.K. Spitznagel, Infect. Immun., 56:1589 (1988)]; (3) BPI Protein competition with polymyxin B (PMB) for binding to S. typhimurium [Parley 1988]; and (4) BPI Protein sequence homology and immunocross-reactivity to another LPS-binding protein Lipopolysaccharide Binding Protein (LBP). LBP-LPS complexes have been shown to stimulate the oxidative burst on neutrophils in response to formulated peptides. In addition, LBP-LPS complexes bind back to a cell surface receptor on monocytes (CD 14) resulting in the induction of tumor necrosis factor (TNF) production. Thus, LBP mediates the immunostimulatory activity of LPS and therefore stimulates the toxic response to endotoxin. High density lipoprotein (HDL), another LPS binding protein found in human serum in complex with LPS, does not show the stimulatory effect on neutrophils.

BPI Protein binding to gram-negative bacteria disrupts LPS structure, alters microbial permeability to small hydrophobic molecules and causes cell death. [Weiss, et al., (1978)]. BPI Protein kills bacteria under physiologic conditions of pH and ionic strength in vitro, indicating that it may be active in vivo outside the low pH environment of the phagolysosome. All of the bactericidal and permeability increasing activities of BPI Protein are present in the N-terminal 25kD fragment of the protein. [Ooi, C.E., J. Weiss, P. Elsbach, B. Frangione, and B. Marrion, J. Biol. Chem., 262: 14891 (1987)]. It was previously understood that the beneficial effects of BPI Protein are limited to its bactericidal effects.

However, it was later shown that BPI Protein also binds to endotoxin but that, unlike LBP, BPI Protein inhibits the immunostimulatory activities of LPS rather than promoting them.

Mannion, et al. [Preferential Binding of the Neutrophil Cytoplasmic Granule-Derived Bactericidal/Permeability Increasing Protein to Target Bacteria, J. Immunol. 142:2807 (1989)] showed that BPI Protein could be purified from neutrophil granule extracts by binding to gram-negative bacteria. Eighty percent of the bound BPI Protein could then be eluted with 200 mM $MgCl_2$, in 10 mM sodium acetate pH 4.0 at 37°C and the bacteria removed by centrifugation.

Expression of many proteins in bacteria, such as E. coli, has proved to be difficult. Which proteins will be expressed in E. coli and whether or not the protein will be expressed in the form of a denatured insoluble inclusion body or folded into active form cannot be determined ahead of time. A variety of conditions can be explored to shift the amount of insoluble vs. soluble material, including fermentation conditions such as media composition, pH, ionic strength, and temperature [A. Mitraki and J. King Bio/Technology 7:690-697, 1989, or C.H Schein Bio/Technology 7: 1141-1149 (1989)].

Further, for proteins in insoluble inclusion body form, a variety of techniques can be explored to recover renatured active protein (BioTechniques 3:298-306 (1985)), but with success very much dependent on the character of the protein in question.

US Patent No. 5,171,739 (Scott and Marra) discloses a method for producing a BPI protein or protein variant thereof from a gram negative bacterial cell which comprises (a) constructing a vector comprising DNA encoding BPI to which no leader peptide sequence is attached; (b) transfecting the cell with the vector; and (c) culturing the cell so transfected in culture medium under conditions such that the BPI protein or protein variant is produced.

The subject invention provides, inter alia, a method for producing Bactericidal/Permeability Increasing Protein, which includes BPI Protein, using gram-negative bacteria. BPI Protein is a protein possessing antibiotic functions, i. e., potent antibacterial activity against gram-negative bacteria. Accordingly, one skilled in the art would not expect to be able to express a protein in active soluble form in a host cell, which protein functions as a known cytotoxin against the host cell. Indeed, the fact that BPI Protein kills gram-negative bacteria has prevented others in the field from developing a bacterial expression system for BPI Protein. The method of the subject invention represents a substantial advance over the state of the art, since the production of recombinant proteins in gram-negative bacteria, such as E. coli, can be up to 100 times more cost effective than other production methods such as mammalian cell expression systems.

## Summary of the Invention

The subject invention provides a method for producing non-glycosylated Bactericidal/Permeability Increasing Protein using a gram-negative bacterial cell, which comprises transforming or transfecting the cell with a vector encoding Bactericidal/Permeability Increasing Protein wherein the Bactericidal/Permeability Increasing Protein has no leader peptide attached thereto when expressed; culturing the cell so transformed or transfected so as to produce non-glycosylated Bactericidal/Permeability Increasing Protein in the cell; and recovering from the cell the non-glycosylated, Bactericidal/Permeability Increasing Protein so produced; characterized in that the cell is cultured at a temperature below 37°C.

In the preferred embodiment, the cell is cultured at a temperature between 25-36°C, most preferably at a temperature of 26°C.

In one embodiment, the recovering comprises the steps of (a) lysing the cell under conditions preventing the formation of a complex between the Bactericidal/Permeability Increasing Protein and LPS so as to release the Bactericidal/Permeability Increasing Protein from the cell; and (b) isolating the Bactericidal/Permeability Increasing Protein so released.

In another embodiment, the recovering comprises the steps of (a) lysing the cell under conditions permitting the formation of a complex between the Bactericidal/Permeability Increasing Protein and LPS; (b) isolating the resulting Bactericidal/ Permeability Increasing Protein-LPS complex; (c) dissociating the Bactericidal/Permeability Increasing Protein-LPS complex so isolated, so as to release the Bactericidal/Permeability Increasing Protein from the Bactericidal/Permeability Increasing Protein-LPS complex; and (d) isolating the Bactericidal/Permeability Increasing Protein so released.

According to another aspect of the present invention, there is provided a method of recovering non-glycosylated Bactericidal/Permeability Increasing Protein from a gram-negative bacterial cell transformed or transfected with a vector encoding Bactericidal/Permeability Increasing Protein having no leader peptide attached thereto when expressed, and cultured so as to produce non-glycosylated Bactericidal/Permeability Increasing Protein in the cell, characterised in that the cell has been cultured at a temperature below 37°C, which method comprises the steps of:

(a) lysing the cell under conditions preventing the formation of a complex between the Bactericidal/Permeability Increasing Protein and LPS so as to release the Bactericidal/Permeability Increasing Protein from the cell; and

(b) isolating the Bactericidal/Permeability Increasing Protein so released.

According to a further aspect of the present invention, there is provided a method of recovering non-glycosolated Bactericidal/Permeability Increasing Protein from a gram-negative bacterial cell transformed or transfected with a vector encoding Bactericidal/Permeability Increasing Protein having no leader peptide attached thereto when expressed, and cultured so as produce non-glycosylated Bactericidal/Permeability Increasing protein in the cell, characterised in that the cell has been cultured at a temperature below 37°C, which method comprises the steps of:

(a) lysing the cell under conditions permitting the formation of a complex between the Bactericidal/Permeability Increasing Protein and LPS;
(b) isolating the resulting Bactericidal/Permeability Increasing Protein-LPS complex;
(c) dissociating the Bactericidal/Permeability Increasing Protein-LPS complex so isolated, so as to release the Bactericidal/Permeability Increasing Protein from the Bactericidal/Permeability Increasing Protein-LPS complex; and
(d) isolating the Bactericidal/Permeability Increasing Protein so released.

Finally, the subject invention discloses the non-glycosylated Bactericidal/Permeability Increasing Protein produced by the method of the subject invention.

## Brief Description of the Figures

### Figure 1

Schematic drawing of cDNA encoding BPI Protein.

### Figure 2

A nucleotide and amino acid sequence of BPI Protein mutagenic primer 25 kDa Pro 212 TGA which is a C-terminal truncation of BPI Protein.

### Figure 3

A nucleotide and amino acid sequence of BPI Protein mutagenic primer 38 kDa Pro 337 TGA which is a C-terminal truncation of BPI Protein.

### Figure 4

A nucleotide and amino acid sequence of BPI Protein mutagenic primer: preferred ATG 5' HindIII which is a C-terminal truncation of BPI Protein.

### Figure 5

A schematic drawing of PSVBPIMDH.

### Figures 6-1 and 6-2

A schematic drawing of pAc373.

### Figure 7

SDS-PAGE analysis of (1) nBPI Protein (#148104), (2) rBPI Protein (#148159), and (3) rBPI Protein (#148179).

### Figures 8A-1 and 8A-2

Amino acid sequence of BPI Protein.

Figures 8B-1 and 8B-2

cDNA sequence of BPI Protein.

Figure 9

Protein sequence for p337.

Figure 10

Protein sequence for p212.

Figure 11

SDS PAGE analysis of BPI Protein produced in mammalian cells and in E. coli, and of BPI Protein obtained through non-recombinant methods. Lanes 1 and 10 are molecular weight markers; lane 3 is BPI Protein produced in CHO cells; lane 5 is naturally derived BPI Protein; lane 7 is BPI Protein produced in E. coli and partially purified.

Figure 12

Assay comparing TNF release in whole blood for BPI Protein produced in CHO cells and E. coli. For the TNF assay, blood is drawn with citrate anticoagulant from healthy donors. BPI Protein is added, then LPS is added. Incubation is carried out for four hours at $37°C$ with 5% $CO_2$. Samples are centrifuged and plasma collected. TNF is assayed using a sandwich assay.

Figure 13

Activity in LAL assay of BPI Protein produced in E. coli.

**Detailed Description of the Invention**

Specifically, the subject invention relates to a method for producing non-glycosylated Bactericidal/Permeability Increasing Protein using a gram-negative bacterial cell, which comprises transfecting the cell with a vector encoding Bactericidal/ Permeability Increasing Protein; culturing the cell so transfected under conditions permitting the production of non-glycosylated Bactericidal/Permeability Increasing Protein in the cell; and recovering from the cell the non-glycosylated, Bactericidal/Permeability Increasing Protein so produced.

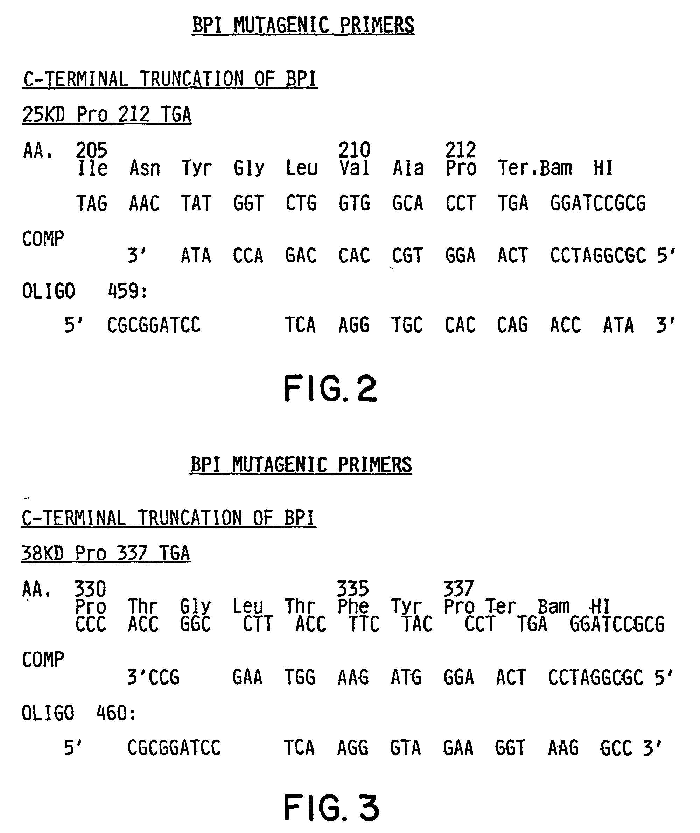

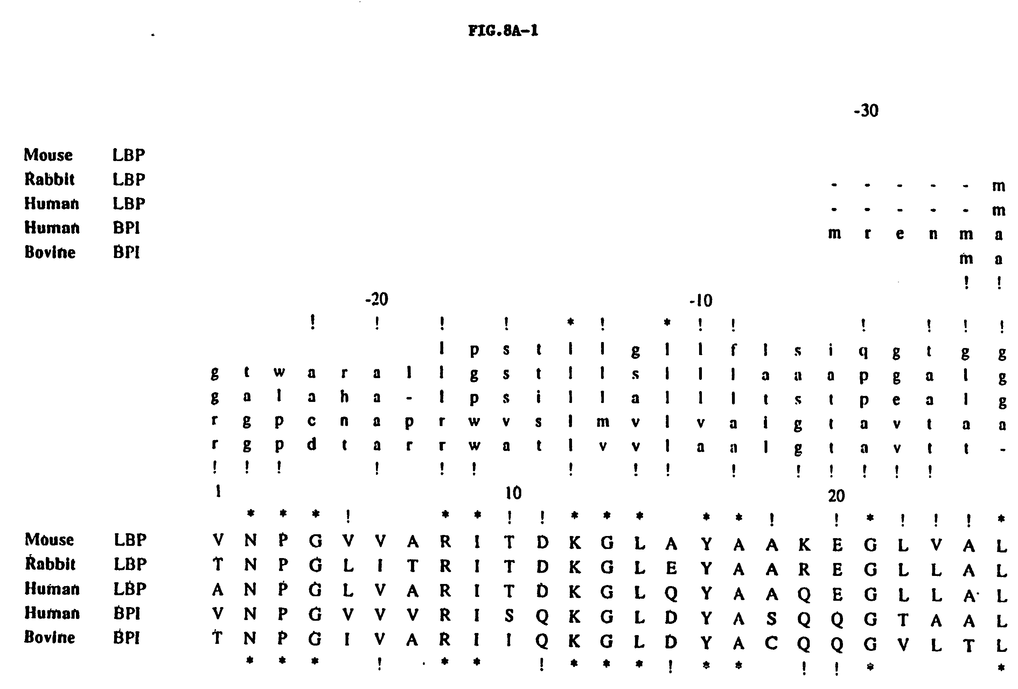

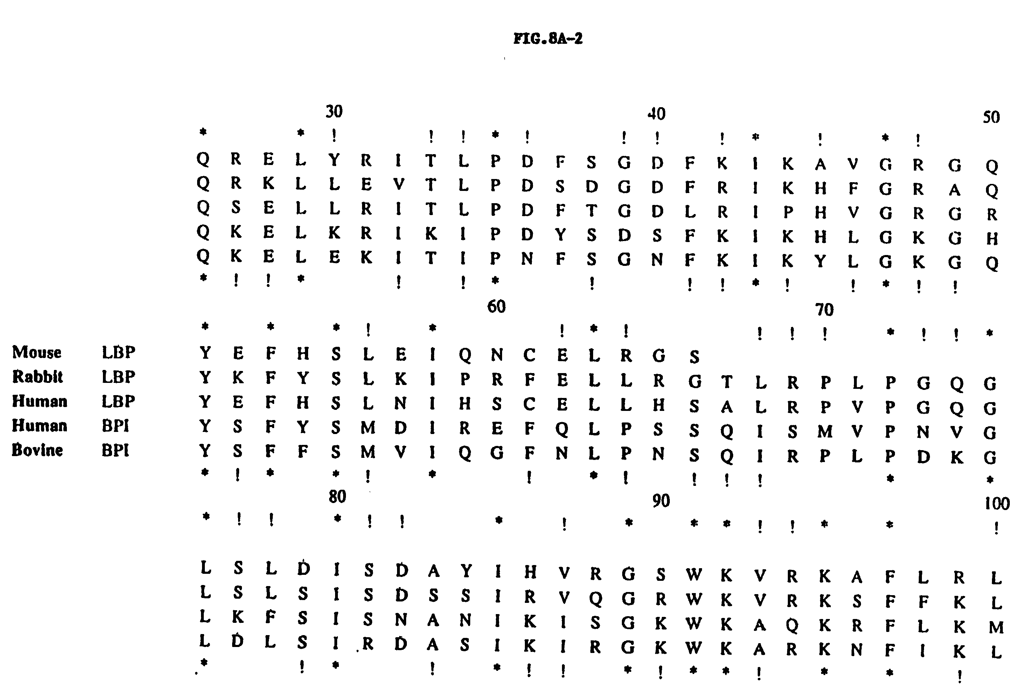

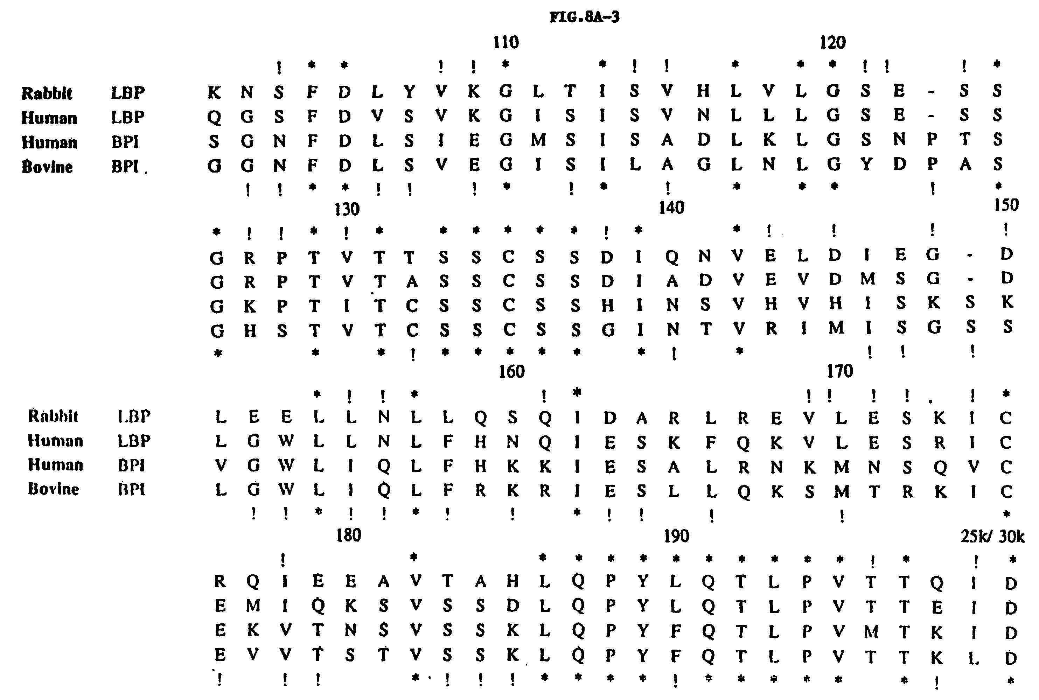

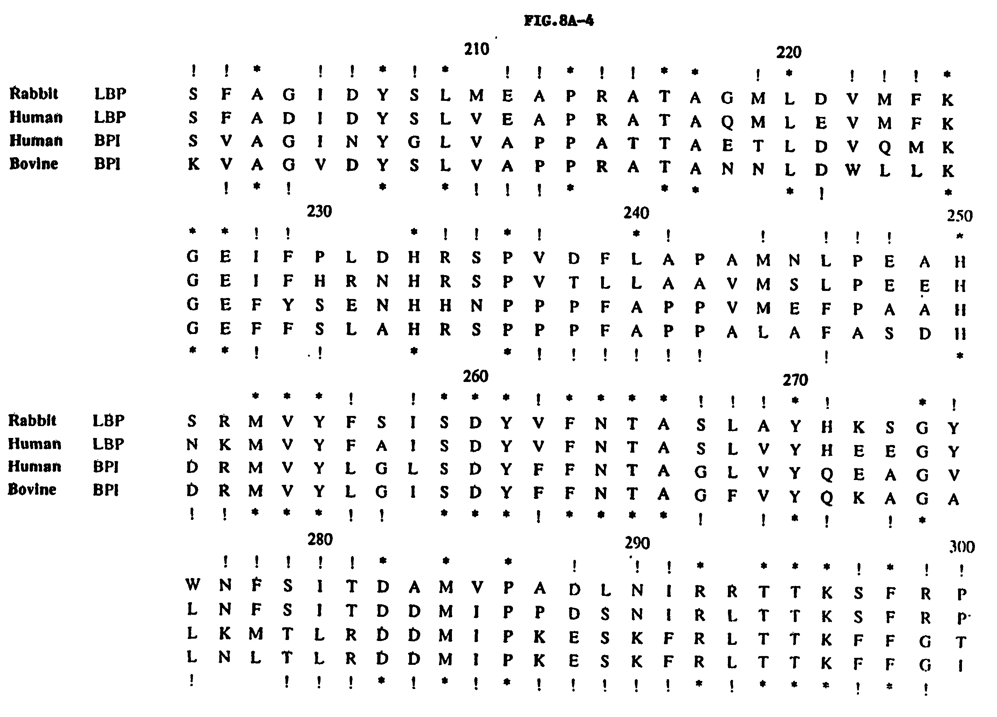

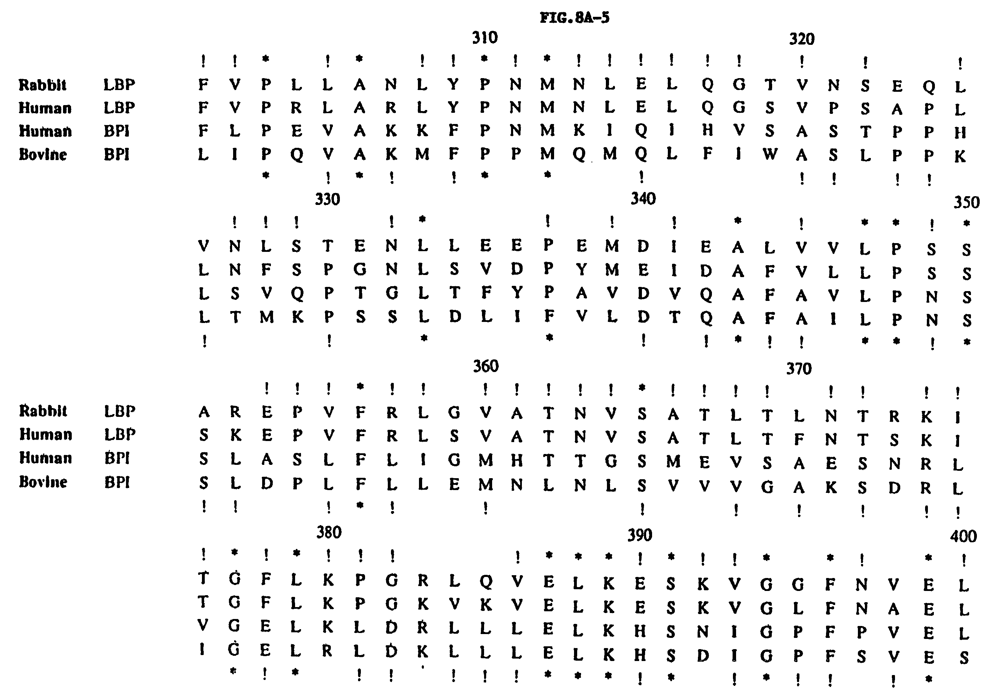

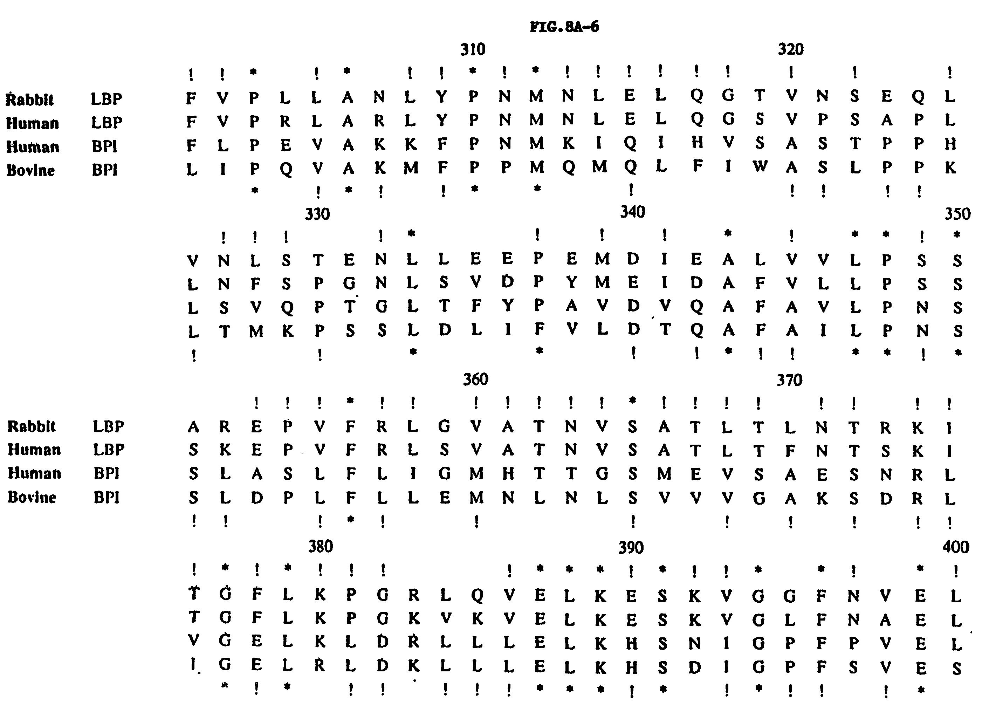

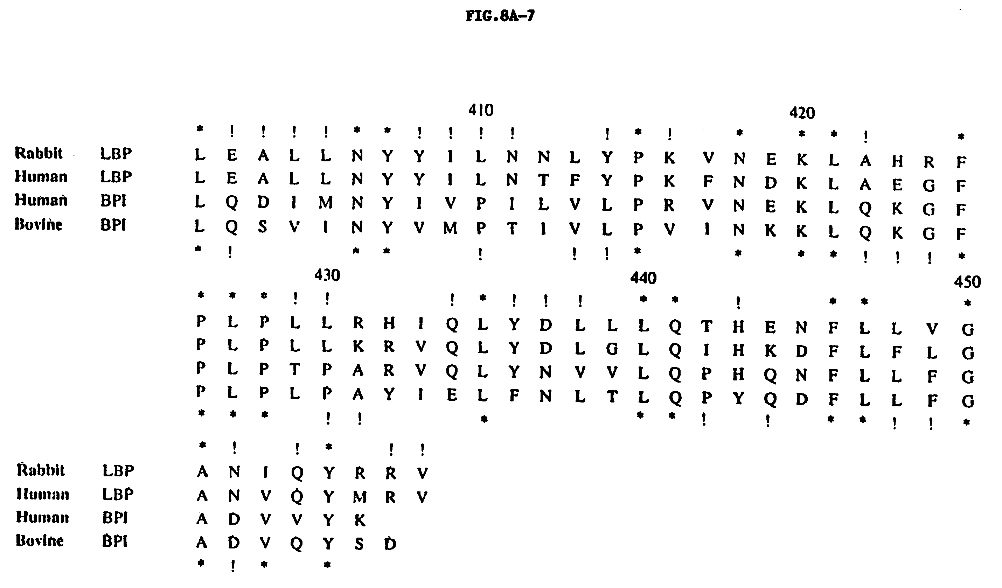

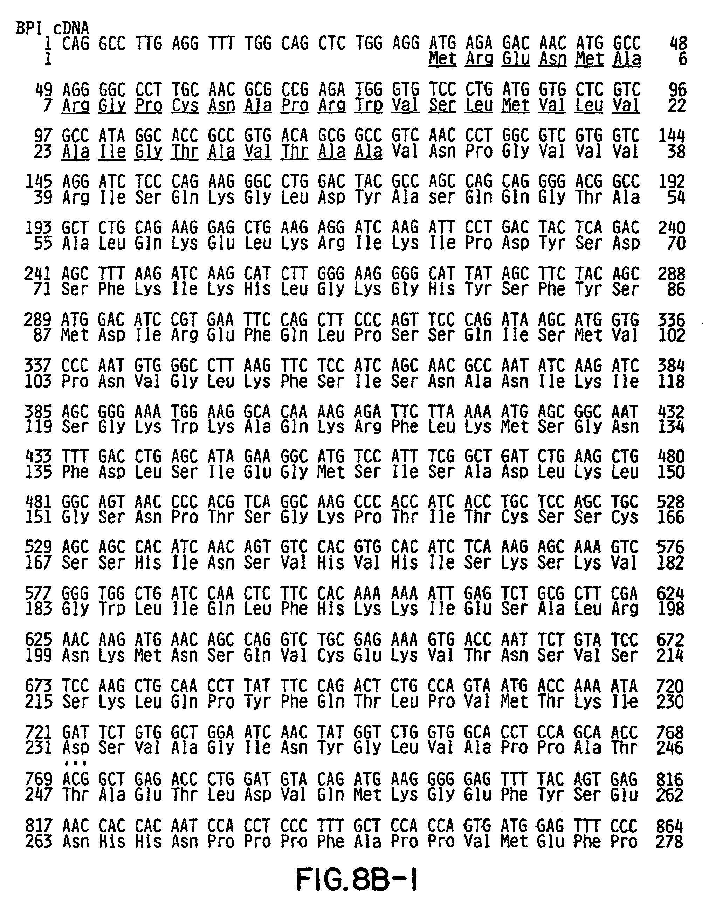

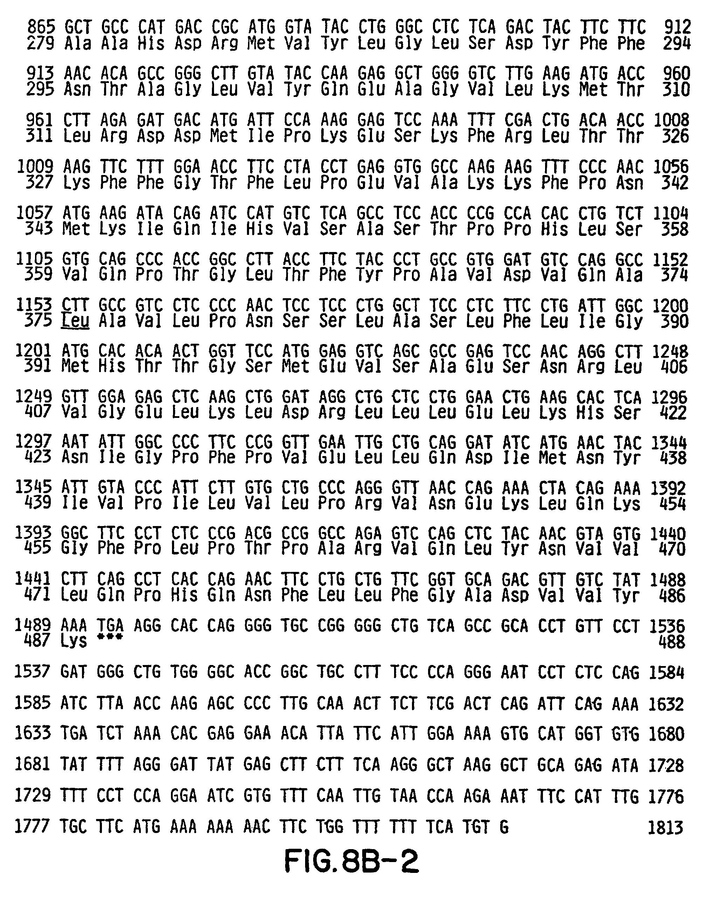

As used herein, Bactericidal/Permeability Increasing Protein means BPI Protein and BPI variants. As used herein, BPI Protein means the native 55 kD human protein having the amino acid sequence shown for human BPI Protein in Figures 8A-1 and 8A-2, and encoded by the cDNA sequence shown in Figures 8B-1 and 8B-2. As used herein, a BPI variant means a protein comprising a portion of BPI Protein, which protein is capable of (a) binding to LPS, (b) competing with BPI Protein or LBP for binding to LPS, and (c) inhibiting the LPS-mediated production of TNF$\alpha$ by human monocytes. BPI variants include, but are in no way limited to, a fragment of BPI Protein, a point mutant of BPI Protein, a deletion mutant of BPI Protein, or a point and deletion mutant of BPI Protein. Specific BPI variants include, but are in no way limited to, $B_{(S351->A)}$ (glycosylation site deleted); $B_{(D200->DP)}$ (acid cleavage site inserted); $B_{1-199}$ (N-terminal domain of BPI Protein); $B_{200-456}$ (C-terminal fragment of BPI Protein); $B_{(F61->C)}$ (cysteine insertion) ; $B_{(C132->A)}$ (cysteine deletion); $B_{(C132->S)}$ (cysteine deletion); $B_{(C135->S)}$ (cysteine deletion); $B_{(C-175->S)}$ (cysteine deletion); $B_{(C132->A)(C135->S)(C175->S)}$ (multiple cysteine deletions); $B_{(C-132->S)(C135->S)(C175->S)}$ (multiple cysteine deletions).

In the BPI variant designations supra, the portion of BPI Protein in the variants is designated by the letter B, followed by an amino acid sequence numbering assignment corresponding to that shown in Figures 8A-1, 8A-2, 8B-1 and 8B-2 for human BPI Protein, wherein the mature N-terminus is designated as residue 1. Single amino acid residue substitutions are noted in parentheses, wherein the original amino acid residue is indicated (using the standard one letter code for amino acids), followed by the substitute amino acid residue. For example, the BPI variant wherein the original serine residue at position 351 is substituted with an alanine residue (which substitution removes a glycosylation signal) is designated $B_{(S351->A)}$. The amino acid substitutions may be a substitution wherein an original amino acid residue at a given position is substituted with the residue at the corresponding position in a different protein. $B_{(Xn->Y)}$ is an example of such a substitution, wherein amino acid residue X at position n in BPI Protein is substituted with residue Y which is found at position n in LBP (or rabbit or bovine LBP). Amino acid residue insertion changes are noted in parentheses, by indicating the amino acid residue after which the insertion occurs, followed by the amino acid residue after which the insertion occurs together with the inserted residue or residues. For example, in $B_{(D199->DP)}$, a proline residue is inserted after the aspartate residue at position 199.

BPI variants having one or more cysteine residues deleted or substituted with serine or another amino acid residues would be useful for preventing the aggregation of BPI variants during their production or use.

The BPI variants may have one or more non-conserved glycosylation sites deleted. Alternatively, the BPI variants may have one or more non-conserved glycosylation sites inserted.

The BPI variants may have one or more secondary structure-altering amino acid residues deleted or added. For example, one or more of the non-conserved proline residues in BPI Protein may be substituted with a non-proline residue.

As used herein, LPS means lipopolysaccharide. As used herein, TNF$\alpha$ means tumor necrosis factor alpha.

In one embodiment, the gram-negative bacterial cell is E. coli.

Methods of transfecting gram-negative cells, vectors suitable for transfecting gram-negative cells, and methods of culturing gram-negative cells are all well known to those skilled in the art. Examples of such methods and vectors are provided infra.

According to the invention, the Bactericidal/Permeability Increasing Protein has no leader peptide attached thereto when expressed. As used herein, the term "when expressed" means when first translated from the mRNA.

In the preferred embodiment, the conditions permitting the production of non-glycosylated Bactericidal/Permeability Increasing Protein in the cell comprise low temperature. As used herein, the term "low temperature" means a temperature between 25-36°C. In the preferred embodiment, low temperature means a temperature of about 26°C.

The Bactericidal/Permeability Increasing Protein may be produced in active soluble form. Two embodiments are provided infra for recovering Bactericidal/Permeability Increasing Protein produced in active soluble form.

In one embodiment, the recovering comprises the steps of (a) lysing the cell under conditions preventing the formation of a complex between the Bactericidal/Permeability Increasing Protein and LPS so as to release the Bactericidal/Permeability Increasing Protein from the cell; and (b) isolating the Bactericidal/Permeability Increasing Protein so released.

In another embodiment, the recovering comprises the steps of (a) lysing the cell under conditions permitting the formation of a complex between the Bactericidal/Permeability Increasing Protein and LPS; (b) isolating the resulting Bactericidal/- Permeability Increasing Protein-LPS complex; (c) dissociating the Bactericidal/Permeability Increasing Protein-LPS complex so isolated, so as to release the Bactericidal/Permeability Increasing Protein from the Bactericidal/Permeability Increasing Protein-LPS complex; and (d) isolating the Bactericidal/Permeability Increasing Protein so released.

In one embodiment lysing the cell comprises removing the outer membrane of the cell, and subsequently breaking open the resulting cell.

In one embodiment, dissociating the Bactericidal/Permeability Increasing Protein-LPS complex comprises contacting the complex with divalent cations. contacting the complex with divalent cations may comprise contacting the complex with 0.05M - 1.0M $MgCl_2$. In the preferred embodiment, contacting the complex with divalent cations comprises contacting the complex with 0.5M $MgCl_2$.

Alternatively, the Bactericidal/Permeability Increasing Protein may be produced in inactive, insoluble form and be stored in inclusion bodies. Disclosed infra is a mechod for recovering Bactericidal/Permeability Increasing Protein produced in inactive, insoluble form.

In this method, the recovering comprises the steps of (a) extracting from the inclusion bodies the Bactericidal/Permeability Increasing Protein in its inactive, insoluble form, and (b) refolding the inactive, insoluble protein so as to generate active, soluble Bactericidal/Permeability Increasing Protein. Examples of this embodiment are provided infra.

Also disclosed is a method for producing non-glycosylated Bactericidal/Permeability Increasing Protein using a prokaryotic cell, which comprises transfecting the cell with a vector encoding Bactericidal/- Permeability Increasing Protein; culturing the cell so transfected under conditions permitting the production of non-glycosylated Bactericidal/Permeability Increasing Protein in the cell; and recovering from the cell the non-glycosylated, Bactericidal/Permeability Increasing Protein so produced. In one example, the prokaryotic cell is a gram-positive cell.

Finally, the subject invention discloses the non-glycosylated Bactericidal/Permeability Increasing Protein produced by the method of the subject invention.

The subject invention is illustrated in the Experimental Details section which follows. This section is set forth to aid in understanding the subject invention, but is not intended to, and should not be construed to, limit in any way the invention as set forth in the claims which follow.

## Experimental Details

## Example 1

## Purification and characterization of Neutrophil BPI Protein

## A. Materials and Methods

### 1. Reagents

Lipopolysaccharide from E. coli 0111:B4, S. typhimurium wild type, glycolipid from S. typhimurium RE mutant, and Lipid A from S. typhimurium RE mutant, and LPS from P. aeruginosa were purchased from RIBI Immmunochem Research, Inc. (Hamilton, MT); Fmet-Leu-Phe (FMLP) and polymyxin B Sulfate were purchased from Sigma Chemical Co. (St. Louis, MO); Hank's Balanced Salt Solution without calcium, magnesium and phenol red (HBSS) were purchased from Hazelton Research Products (Denver, PA); Ficoll-Paque, Percoll and Macrodex were purchased from Pharmacia, Inc. (Piscataway, NJ); TNF and anti-TNF were purchased from Endogen (Boston, MA); Fluorescein-conjugated goat anti-mouse IgG was purchased from TAGO, Inc. (Burlingame, CA); IgG1 control antibody was purchased from Coulter Immunology (Hialeah, FL); Phycoerythrin (PE) conjugated anti-CR3 (Leu-15) and IgG2a control were purchased from Becton Dickinson (Mountain View, CA); and anti-CR1 monoclonal antibodies may be obtained commercially or according to methods known to those skilled in the art.

### 2. Azurophil Granule Isolation and Extraction

Granulocytes were isolated from buffy coats obtained from local blood banks. Buffy coats were diluted 3-4X in HBSS and granulocytes were separated from mononuclear cells by centrifugation through 64% Percoll. The pellet was subjected to diisopropylfluorophosphate (DFP), washed, and resuspended in ice cold lysis buffer (10 mM PIPES, pH 6.8, 100 mM KCl, 3mM NaCl, 3.5 mM MgCl2) and disrupted by nitrogen cavitation (Parr Instrument Co., Moline, IL). Azurophil granules were isolated on discontinuous Percoll gradients as described by Borregaard. [Borregaard, N., J.M. Heiple, E.R. Simons, and R.A. Clark, J. Cell. Biol., 97: 52-61 (1983)]. The azurophil granules were collected and Percoll was removed by centrifugation at 180,000 X g for 2 hours. The granules were lysed by 4 cycles of freeze-thaw followed by 1 minute of sonication. The lysed granules were extracted in an equal volume of 100 mM glycine, pH 2 by vortexing intermittently for 1 hour at room temperature. The acid extract was clarified by centrifugation at 30,000 X g for 20 minutes and at 200,000 X g for 30 minutes.

### 3. Neutrophil Isolation

Venous blood was drawn from healthy volunteer donors into acid citrate dextrose anticoagulant and immediately placed on ice. Five parts blood were mixed with 1 part cold Macrodex, and allowed to settle for 1.5 - 2 hours at 4°C. Leukocyte-rich plasma was washed 1X in cold HBSS, then resuspended in HBSS and layered over Ficoll-Paque. If significant erythrocyte contamination was present, the granulocyte pellet was subjected to hypotonic lysis. The cells were washed 2X in HBSS and resuspended in HBSS + 2% autologous plasma to give a final granulocyte concentration of 1 X 10$^6$/ml in the incubation mixture.

### 4. BPI Protein Purification

Approximately 2 mg of crude azurophil granule extract was separated by size on a Biosil (TSK-250) (7.8 mm x 600 mm) high performance size exclusion column using 50 mM glycine and 100 mM NaCl buffer, pH 2.0, under isocratic conditions of a flow rate of 1 ml/min. Column fractions with the greatest LPS inhibitory activity contained a large proportion of the 54 kD species as shown by SDS PAGE. These TSK fractions were pooled and run over an Aquapore weak cation exchange (WCX) column (2.1 mm X 30 mm) using 50 mM citrate, pH 5.5, and eluted in a gradient of 0-75%, of 50 mM citrate and 1 M NaCl (Buffer B) in 25 min, then 75-100% Buffer B in 5 min with a flow rate of 200 ml/min. Material of 55 kD was recovered from cation exchange and appeared as a single band on SDS page. In some experiments BPI Protein was further purified by reverse phase HPLC on a Vydac C4 column loaded for 12 min in 0.1% CH$_3$CN plus 0.1% TFA, in 30 min with a flow rate of 200 ml/min (Rainin Instruments, Emeryville, CA).

### 5. Neutrophil stimulation

Isolated neutrophils were kept on ice until incubated with and without stimuli at 37°C for 30 minutes. Following the incubation, cells were washed in a large volume of cold PBS + 0.05% Na Azide + 2% autologous plasma. Pellets were divided in two, one stained with 50 μl control IgG1 antibody (20 μg/1 x $10^6$ cells), the other with 50 μl of 20 μg/1 x $10^6$ cells anti-CR1 for 30 minutes at 0°C. Following this incubation the cells were washed 2X with PBS + autologous plasma, then stained with goat-anti-mouse IgG-FITC, and in some experiments, 20 μl of IgG2a-phycoerythrin (PE) in control wells, and 20 μl Leu-15 PE in test wells. Following a 30 minute incubation at 0°C and 2 more washes, the cells were analyzed by flow cytometry on a Becton Dickinson FACStar flow cytometer (Becton Dickinson, Mountain View, CA). Neutrophil stimulation was measured by comparing mean fluorescence intensity of samples which had been incubated in HBSS + 2% autologous plasma alone (control) to those incubated with LPS or LPS which had been preincubated for 30 minutes at 37°C with BPI Protein or polymyxin B. Data are expressed as % stimulation or % inhibition and were calculated using the mean fluorescence intensity (F1), on a log scale, according to:

$$\% \text{ Stimulation} = [(\text{Experimental} - \text{Control})/(\text{Maximum} - \text{control})] \text{ X } 100$$

and

$$\% \text{ Inhibition} = 1-[(+\text{Inhibitor})-(\text{Control})]/[(-\text{Inhibitor})-(\text{Control})] \text{ X } 100.$$

6. Amino Acid Analysis

Vapor phase hydrolysis of BPI Protein and amino acid derivitization was performed using a Pico-tag Workstation (Waters, Milford MA) and chromatographic analysis of the phenylthiocarbamyl amino acids was performed on an applied Biosystems 130 A MPLC using Protocols provided by the manufacturer.

7. Sequence Analysis

BPI Protein N-terminal sequence was analyzed by automated Edman degradation using an applied Biosystems 477A pulse liquid phase sequenator (Applied Biosystems, Foster City, CA). Phenylthiohydantoin amino acid analysis was performed on line using an applied biosystems Model 120A liquid chromatograph.

**B. Results**

Human neutrophils may be stimulated both in vivo and in vitro by lipopolysaccharide. Upon activation, surface expression of receptors for C3b and C3bi (CR1 and CR3, respectively) increases. Using the Fluorescence Activated Cell Sorter (FACS), fluorescence intensity of freshly isolated human neutrophils was measured following stimulation with increasing doses of 0111:B4 LPS (data not shown). Because commonly observed maximum stimulation was at or above 10 ng/ml, experiments testing for inhibition of 0111:B4 LPS used 10 ng/ml as the stimulatory dose. All experiments were performed in duplicate. In most experiments, data is shown only for CR1 since no conditions were observed wherein neutrophil stimulation caused upregulation of CR1 or CR3 alone [M. Marra et al., J. Immunol. 144(2):662-666 (1990)].

To determine whether proteins found in neutrophil azurophil granules could interfere with the neutrophil response to LPS, crude acid extracts of azurophil granules were pre-incubated with LPS for 30 minutes at 37°C. The mixture was then tested for its ability to stimulate neutrophils. Azurophil protein (1 μg/ml) could effectively block stimulation of 1 X $10^6$ polymorphonuclear leukocytes (PMN)/ml by 10 ng/ml of LPS (data not shown). This effect was not observed using glycine extraction buffer preincubated with LPS, nor was there any stimulation of neutrophils using crude extract or glycine buffer control (data not shown).

To further investigate which of the proteins in the extract was/were responsible for inhibitory effect, crude acid extracts were separated by reverse phase HPLC, and each peak was assayed separately for LPS inhibitory activity. The identity of each of the peaks was previously determined using a two-dimensional purification approach involving microbore reverse phase HPLC in the first dimension followed by SDS PAGE, electroblotting and microsequencing. The azurophil proteins can be resolved into 10 discrete peaks whose identities are shown in Table 1. The amino acid sequences shown are for the first 15 amino acids of the N-terminal.

LPS inhibitory activity of 1 μg of each peak was tested. Peak 9 had the highest LPS neutralizing activity. The major protein species in this peak has N-terminal identity with BPI Protein described previously [Weiss, J., P. Elsbach, I. Olsson and H. Odeberg, J. Biol. Chem, 253 (8):2664-2672 (1978)]. BPI Protein has been shown to contain the majority of the gram-negative bactericidal activity in azurophil granule protein extracts. Cathepsin G showed some inhibition of LPS, but the data between experiments were not as reproducible as for peak 9. Cathepsin G has been shown to bind to LPS in vitro and to kill gram-negative organisms, although to a lesser extent than BPI Protein. Other proteins which have demonstrated microbicidal activity against gram-negative organisms are elastase and the defensins. However, these proteins (1 μg/ml) did not inhibit the stimulatory activity of LPS on neutrophils.

LPS inhibitory activity of crude azurophil extracts was further characterized and purified using size exclusion and ion exchange followed by reverse phase chromatography. LPS inhibitory activity comigrates with a pure 55 kD band seen on SDS PAGE (data not shown).

Because the buffer used in the RPLC separations ($CH_3CN$ and 0.1% trifluoroacetic acid (TFA)) significantly diminishes the LPS inhibitory activity of BPI Protein (data not shown), and since the material purified from ion exchange chromatography was of high purity as judged by SDS PAGE, size exclusion/ion exchange material was used to generate a dose response curve.

## Table 1

### Azurophil Granule-Derived Proteins

| Peak | Identity | 1 | | | | 5 | | | | 10 | | | | 15 |
|------|----------|---|---|---|---|---|---|---|---|----|---|---|---|----|
| 1 | Defensins (HNP-2) | C | Y | C | R | I | P | A | C | I | A | G | E | R R Y |
| 2 | Granulocidin (HNP-4) | V | C | S | C | R | L | V | F | C | R | R | T | G L R |
| 3 | Eosinophil Cationic Protein (ECP) | X | P | P | Q | F | T | R | A | Q | W | F | A | I Q H |
| 4a | Eosinophil-Derived Neurotoxin (EDN) | K | P | P | Q | F | T | X | A | Q | X | F | E | T Q X |
| 4b | Cathepsin G | I | I | G | G | R | E | S | R | P | H | S | R | P Y M |
| 5a | Lysozyme | K | V | F | E | R | X | E | L | A | R | T | L | K R L |
| 5b | Eosinophil Major Protein (MBP) | T | C | R | Y | L | L | V | R | S | L | Q | T | F S Q |
| 6 | Unknown | I | V | G | G | R | K | A | R | P | X | Q | F | P F L |
| 7 | Unknown | I | V | G | G | H | E | A | Q | P | H | S | R | P Y M |
| 8a | Myeloperoxidase | V | N | C | E | T | S | C | V | Q | Q | P | P | C F P |
| 8b | Elastase | I | V | G | G | R | R | A | R | P | H | A | X | P F M |
| 9 | BPI Protein | V | N | P | G | V | V | V | R | I | S | Q | K | G L D |

This size exclusion/ion exchange purified material was confirmed to be BPI Protein by N-terminal sequence analysis. Protein concentration was determined by amino acid analysis. About 90 ng/ml of BPI Protein is required for maximal inhibition of the neutrophil response to 10 ng/ml 0111/B4 LPS. The neutrophil response to formulated peptide ($10^{-7}$ M FMLP) was not inhibited by BPI Protein (data not shown).

A similar dose response curve for the polypeptide antibiotic Polymyxin B (PMB) was seen. Polymyxin B binds to the Lipid A moiety of LPS and neutralizes some of its toxic effects both in vivo and in vitro. Polymyxin B binds to LPS stoichiometrically [Morrison, D.C. and D.M. Jacobs, Immunochem, 13: 813-818 (1976)]. The calculated amount of PMB required to inhibit 10 ng/ml of smooth LPS is approximately 0.67 nM. In the subject experiments 0.4 ng/ml, or 0.36 nM of polymyxin B was required to completely inhibit neutrophil stimulation using 10 ng/ml of LPS. 90 ng/ml, or 1.58 nM BPI Protein was required for 100% inhibition of 10 ng/ml LPS.

Therefore, on a molar basis the amount of BPI Protein required to inhibit LPS stimulation of neutrophils in vitro was approximately 4 times the amount required for polymyxin B.

To test whether BPI Protein can inhibit LPS from other gram-negative organisms, LPS molecules with varying polysaccharide chain lengths and Lipid A were tested in the subject system (CR1 stimulation in neutrophils) against 90 ng/ml of 2X purified BPI Protein. Data shown in Table 2 demonstrate that the stimulatory dose may vary between these molecules and that LPS from both smooth and rough chemotypes as well as Lipid A are all inhibited by BPI Protein.

Table 2

| Effect of BPI Protein on Neutrophil Stimulation by Various Agents | | |
|---|---|---|
| Inhibition of CR Upregulation on Neutrophils | | |
| LPS | 10 ng/ml | 1 ng/ml |
| E.COLI 0111:B4 | 97 | * |
| S. TYPHIMURIUM WILD TYPE | 103 | 113 |
| S. TYPHIMURIUM RE MUTANT | 113 | 109 |
| S. TYPHIMURIUM RE MUTANT LIPID A | 33 | 99 |
| P. AERUGINOSA | 112 | * |

\* Low to no stimulation at this endotoxin concentration

## Example 2

### Preliminary Studies On Neutrophil BPI Protein

In response to LPS, human neutrophils upregulate cell surface expression of complement receptors CR1 and CR3 (data not shown). To measure this neutrophil response to LPS, freshly isolated human neutrophils were incubated with E. coli 0111:B4 LPS (data not shown), and showed that maximal CR1 upregulation is observed using 10 ng/ml LPS (data not shown). Neutrophil stimulation with LPS was not inhibited by exogenous anti-TNF antibodies, suggesting that LPS acted directly on neutrophils in this system.

BPI Protein inhibits the neutrophil response to LPS (data not shown). Inhibition of CR upregulation was complete at a dose of approximately 1.8-3.6 nM (100-200 ng/ml) BPI Protein, compared to 0.4 nM polymyxin B required to inhibit 10 ng/ml smooth LPS (approximate m.w. 15,000) which is about 0.7 nM, matching closely with the observed value of 0.4 nM. On a molar basis, the amount of BPI Protein required to inhibit LPS was approximately 5-fold greater than the amount required for polymyxin B.

BPI Protein inhibits LPS-mediated neutrophil stimulation but not stimulation by either FMLP or TNF (Table 3). These data demonstrate that BPI Protein inhibits LPS directly and does not disrupt neutrophil mechanisms involved in CR upregulation.

Table 3

| Effect of BPI Protein on Neutrophil Stimulation by Various Agents | | | |
|---|---|---|---|
| Inhibition of CR Upregulation on Neutrophils | | | |
| Stimulus | Dose | % Inhibition CR1 | % Inhibition CR3 |
| LPS | 10 ng/ml | 109 | 102 |
| FMLP | $10^{-7}$ M | 9 | 11 |
| rTNF | 50 U/ml | 0 | 0 |
| Neutrophils were incubated with E. coli 0111:B4 LPS, FMLP or TNF preincubated in the presence or absence of 2.7 nM BPI Protein. Data is reported as percent inhibition of CR expression in response to each stimulus preincubated with buffer alone. Neutralization of LPS by BPI Protein occurred rapidly. Even without preincubation, both BPI Protein (and polymyxin B) inhibited more than 70% of the neutrophil response to LPS (data not shown). Maximal inhibition was seen following only 5 minutes of preincubation. | | | |

BPI Protein inhibits CR upregulation stimulated by LPS from smooth and rough bacterial strains, as well as lipid A (Table 4). Because of the broad range of BPI Protein activity against these different forms of LPS, among which only lipid A and 2-keto-3-deoxy-octonate are shared determinants, it is likely that LPS inhibition by BPI Protein is affected through lipid A.

Table 4

| Inhibition of LPS and Lipid A-induced Neutrophil Stimulation by BPI Protein | | | |
|---|---|---|---|
| Inhibition of CR Upregulation on Neutrophils | | | |
| Stimulus | Dose (ng/ml) | CR1 % Inhibition | CR3 %Inhibition |
| None | - | 0 | 0 |
| E. Coli 011:B4 LPS | 10 | 100 | 99 |
| S. typhimurium Wild Type LPS | 10 | 104 | 100 |
| S. typhimurium RE Mutant LPS | 1 | 97 | 95 |
| S. typhimurium RE Mutant Lipid A | 1 | 111 | 104 |
| Neutrophils were stimulated with LPS and lipid A preincubated with and without 2.7 nM purified BPI Protein. Results are expressed as percent inhibition of fluorescence intensity observed with each type of LPS alone. | | | |

BPI Protein inhibits other LPS-mediated activities. At a concentration of approximately 9 nM, or 500 ng/ml, BPI Protein significantly inhibited LPS activity in the LAL assay (data not shown). When LPS and BPI Protein were added together without preincubation, no inhibition was observed (data not shown), indicating that BPI Protein acted on LPS, and had no effect on the LAL assay system. BPI Protein also inhibits LPS-mediated TNF production by human adherent mononuclear cells (Table 5).

Table 5

| BPI Protein Inhibits LPS-Induced TNF Production by Human Monocytes | | | | | |
|---|---|---|---|---|---|
| TNF (pg/ml) Produced in Response to LPS Preincubated with*: | | | | | |
| LPS | Medium | 100 ng/ml | 500 ng/ml | 250 ng/ml | Buffer |
| (ng/ml) | Alone | | Polymyxin B | BPI | BPI Control |
| 0 | 0 | 0 | 0 | 0 | 0 |
| 0.1 | 61 | 0 | 0 | 0 | 81 |

Table 5   (continued)

| BPI Protein Inhibits LPS-Induced TNF Production by Human Monocytes | | | | | |
|---|---|---|---|---|---|
| TNF (pg/ml) Produced in Response to LPS Preincubated with*: | | | | | |
| (ng/ml) | Alone | | Polymyxin B | BPI | BPI Control |
| 1 | 1051 | 96 | 0 | 0 | 1090 |
| 10 | 2053 | 2154 | 1490 | 1746 | 2325 |

*E. coli 0111:B4 LPS, was preincubated with BPI Protein or polymyxin B (PMB), than added to adherent peripheral blood mononuclear cells. TNF production was assayed by ELISA.

BPI Protein was first purified by Elsbach and Weiss in 1978. In the initial studies, BPI Protein was isolated from azurophil granule extracts in a single step by reverse phase HPLC. Recovery of BPI Protein activity from reverse phase was poor, probably due to the denaturing conditions. Here, the purification of LPS inhibitory activity using only non-denaturing steps is shown, and it is demonstrated that most of the activity from neutrophils comigrates with BPI. Improvements in the purification have also led to very high specific activity material as will be shown in the following section.

## Example 3

## Purification of neutrophil BPI Protein under rigorously pyrogen-free conditions

### A. Materials and Methods

1. Reagents

USP grade sterile irrigation water was obtained from Travenol Laboratories Inc. (Deerfield IL); Pyrosart filters were obtained from Sartorius GmbH (Germany); CM Sepharose FF was obtained from Pharmacia (Upsala, Sweden); Polyaspartamide weak cation exchange HPLC column (100 X 4.6mm) was obtained from the Nest Group (Southborough, MA); Glycine and Bio-Sil G250 size exclusion HPLC column (600 X 7.5mm) were obtained from Bio-Rad Laboratories (Richmond, CA); Polyacrylamide electrophoresis gels were obtained from Novex (Encitas, CA); Sequencing and amino acid analysis reagents and buffers were obtained from Applied Biosystems, Inc. (Foster City, CA); Trifluoroacetic acid, constant boiling HCL, hydrolyzate amino acid standard, and BCA protein assay reagents were obtained from Pierce Chemical Co. (Rockford, IL); Limulus Amebocyte Lysate assay was obtained from Whittaker Bioproducts, Inc. (Walkersville, MD); Lipopolysaccharide was obtained from RIBI Immunochem Research, Inc. (Hamilton, MT); HPLC grade Acetonitrile from J.T. Baker (Phillipsburg, NJ); all other buffers and salts used were reagent grade. 18 megohm purity water was prepared by Lab Five ultrapure water system from Technic (Seattle, WA). 0.5M NaOH for sanitization was prepared from reagent grade NaOH pellets and USP water.

2. Granule extracts from Neutrophils

These were prepared as described (U.S. Serial No. 07/199,206, filed May 26, 1988) except that the percoll separation of azurophil granules was omitted. Instead, whole granule fractions were obtained by centrifuging the post nuclear supernatant at 17,000 x g for 20 minutes. The granule pellet was then suspended in a volume of 1 ml of 50 mM glycine, pH 2 for every 4 X $10^8$ cells lysed. Resuspended granules were lysed by five freeze/thaw cycles on dry ice ethanol followed by vigorous agitation for one hour at 4°C. The soluble extract was obtained by centrifugation at 30,000 x g for 30 minutes.

3. Limulus Amebocyte Lysate assay

This assay was performed as directed by the manufacturer. Where necessary the pH of samples was adjusted to neutrality by the addition of pyrogen-free 0.5M phosphate buffer, pH 7.4, and salinity was decreased to <150 mM by dilution with USP water [Desch, C.E., et al. (1989) Infection and Immunity, 57:1612-1614; Friberger, P. (1985)]. The design of a reliable endotoxin test. p. 139-149. In J.W. Cape (ed.). Bacterial endotoxins: structure, biomedical significance and direction with the Limulus amebocyte lysate test. Alan R. Liss, Inc., New York; Galanos, C., et al. (1979) Chemical, physiochemical and biologic properties of bacterial lipopolysaccharides, p. 321-332. In E. Cohen (ed.) Biomedical application of the horseshoe crab (Limulidae). Alan R. Liss, Inc., New York; Byaston, K.F. and J. Cohen (1990) J.Med. Microbiol., 31:73-83; Tanaka, D., et al. (1982) Biochem. Biophys. Res. Comm., 105:717-723; Morita, T., et al.

(1985) J. Biochem., 97:1611-1620].

4. LPS neutralization assay

This assay was performed as previously described [M. Marra et al. (1990) J. Immunol. 144(2):662-666].

5. High salt fractionation of granule extracts

200 mgs of extracted protein were pooled from various preparations and kept on ice. 1 volume of sterile 5M NaCl was added for every 4 volumes of extract. The resulting precipitate was pelleted by centrifugation at 20,000 x g for 20 minutes at 4°C. This supernatant was prepared for CM sepharose chromatography by diluting with 4 volumes of USP irrigation water and adjusting the pH with enough 1M Tris pH 7.4 to give a final concentration of 50 mM. Only fresh, sterile, pyrogen-free stock salts and buffers were used.

6. CM Sepharose chromatography

An XK-16 column (Pharmacia) was packed with sufficient resin to give a bed volume of 5 mls. The column was installed on a gradient FPLC equipped with a P1 pump for sample loading. Prior to use, all surfaces in contact with the mobile phase were extensively washed with 0.5M NaOH. The column was sanitized by washing at 0.2 mls/ min. with 0.5M NaOH for 4 hrs. The column was then re-equilibrated and a blank run was performed. Fractions from the blank run and eluents were tested by LAL assay for pyrogenicity. Prepared extract was loaded at a flow rate of 400 mls/hr. Once loaded the column was washed with 2 to 3 column volumes of starting buffer. The granule extract was kept on ice during loading. The column was run at room temperature.

7. Weak cation exchange HPLC

Weak cation exchange HPLC was performed using an Eldex ternary gradient pump equipped with a Rheodyne injector and a Gilson model 111B U.V. detector. Wettable surfaces were washed with 0.5M NaOH followed by extensive rinsing with USP water to remove all traces of base prior to installing the column. Blank fractions and eluents were tested for pyrogenicity as above.

8. Gel permeation HPLC

Gel permeation HPLC was performed with the same precautions and equipment outlined for weak cation exchange HPLC, except that the TDK column was not treated with NaOH.

9. Polyacrylamide gel electrophoresis

8 to 16% acrylamide gradient gels were purchased from Novex and run according to the manufacturer's specifications.

10. Protein sequence determination

An Applied Biosystems 477A pulsed liquid phase sequenator equipped with a 120A PTH amino acid analyzer was used for automated Edman degradation.

11. Microbore reverse phase HPLC

Material for protein sequencing was prepared by desalting on a 30 X 2.1 mm Aquapore butyl column. Solution A was 0.1% T.F.A. in water and solution B was 70% acetonitrile in water with 0.1% T.F.A. The gradient used was 30 to 100% B in 30 minutes at a flow rate of 200 $\mu$l/minute. Detector settings were 214 nm wavelength at 2.0 absorbance units full scale. An HP 3396A was used to integrate and plot data.

12. Amino Acid Analysis

Amino acid analysis was performed on the system described above using the PTC column, buffers and separation conditions provided by ABI. Sample hydrolysis and PTC derivatives were prepared using a Pico-Tag workstation from the Waters chromatography division of Millipore using the manufacturer's protocols.

13. Protein assays

Protein concentrations were determined using BCA method instructions 23230, 23225 from Pierce Chemical Co. In order to minimize buffer interference, samples were diluted 10-fold and the micro reagent protocol was used.

B. Results

BPI Protein purified from azurophil granules was previously shown to inhibit neutrophil activation by LPS and to inhibit LPS directly in the LAL assay. In order to further define the role of BPI Protein and investigate the presence of other similar molecules in both azurophil and specific granules, the purification of LPS inhibitory activity was undertaken from whole granules extracted at acidic pH. Preliminary studies verified the presence of LPS inhibitory activity in the crude extract.

To identify the endotoxin-neutralizing activity, its purification was attempted from whole granule extracts. Purification of LPS neutralizing activity was greatly enhanced by the observation that high concentrations of NaCl (1M) caused the reversible precipitation of about ninety percent of the protein present in the granule extract. Essentially all of the LPS inhibitory activity remained in the soluble supernatant. The soluble fraction was then diluted, to reduce the ionic strength, and further purified and concentrated by CM sepharose cation exchange chromatography. A broad peak of activity eluted which was subsequently further purified using a polyaspartamide high performance cation exchange column. A somewhat sharper peak of activity was recovered which co-migrated with a major protein of about 55,000 molecular weight by SDS-PAGE along with several lower molecular weight proteins. Gel permeation HPLC was used as the final purification step and identified a peak of activity which eluted with a single sharp protein peak. The purified protein migrated as two closely spaced bands on SDS-PAGE at 55,000 molecular weight representing a mixture of glycosylated and nonglycosylated species. 25% of the total endotoxin-neutralizing activity was recovered with a 250-fold purification.

The purified endotoxin-neutralizing protein was subjected to reverse phase HPLC followed by N-terminal sequence analysis by automated Edman degradation. The sequence was identified as bacterial permeability increasing protein by virtue of complete homology through 39 residues. In addition the amino acid composition of the purified molecule was virtually identical to that of BPI Protein (data not shown).

To investigate whether both closely spaced bands represented BPI Protein, the purified proteins were subjected to Western blotting analysis using BPI Protein-specific rabbit polyclonal antisera raised against a synthetic peptide comprising amino acids 1-20 of BPI Protein. Both bands were immunoreactive. The differences likely arise from glycosylation.

**Example 4**

**LPS inhibitory activities of BPI Protein in vitro**

Purification of BPI Protein under rigorously pyrogen-free conditions, as described, supra, resulted in a more potent BPI Protein preparation. Inhibition of LPS-mediated CR upregulation was complete at 25 ng/ml BPI Protein, representing a 4-fold increase in activity compared to the material used in section I. On a molar basis this BPI Protein preparation inhibited LPS at approximately stoichiometric proportions, equivalent to molar inhibitory concentrations of polymyxin B. BPI Protein also inhibited LPS-mediated TNF production by human adherent mononuclear cells at a lower concentration following purification under pyrogen-free conditions (Tables 7 and 8).

BPI Protein binds to LPS (data not shown). In these experiments, 4 μg of LPS/well was immobilized on 96 well plastic plates, then incubated with varying concentrations of BPI Protein, and developed with anti-BPI Protein polyclonal antisera. BPI Protein binding to LPS was inhibited by polymyxin B (data not shown), demonstrating specificity of BPI Protein binding. BPI Protein binds to LPS in the presence of both plasma (data not shown) and serum (data not shown), demonstrating potential in vivo efficacy of BPI Protein.

Table 7

| BPI Protein Inhibits LPS-Induced TNF Production by Human Monocytes | | | | | | |
|---|---|---|---|---|---|---|
| TNF (pg/ml) Produced in Response to LPS Preincubated with*: | | | | | | |
| LPS ng/ml | Medium alone | 100 ng/ml PMB | 400 ng/ml BPI | 150 ng/ml BPI | 25 ng/ml BPI | Buffer Control |
| 0 | 0 | 0 | 0 | 0 | 0 | 0 |
| 0.1 | 98 | 79 | 0 | 0 | 0 | 269 |

Table 7

| BPI Protein Inhibits LPS-Induced TNF Production by Human Monocytes | | | | | | |
|---|---|---|---|---|---|---|
| TNF (pg/ml) Produced in Response to LPS Preincubated with*: | | | | | | |
| LPS ng/ml | Medium alone | 100 ng/ml PMB | 400 ng/ml BPI | 150 ng/ml BPI | 25 ng/ml BPI | Buffer Control |
| 1 | 1150 | 1207 | 0 | 0 | 0 | 1292 |
| 10 | 1370 | 1270 | 145 | 353 | 559 | 1413 |

*E. coli 0111:B4 LPS, was preincubated with BPI Protein or polymyxin B (PMB), then added to adherent peripheral blood mononuclear cells. TNF production was assayed by ELISA.

Table 8

| Inhibition of LPS-Induced TNF Production by Human Monocytes | | | | | | |
|---|---|---|---|---|---|---|
| TNF (pg/ml) Produced in Response to LPS Preincubated with*: | | | | | | |
| LPS | 1000 ng/ml Polymyxin B | 100 ng/ml Polymyxin B | 250 ng/ml BPI | 50 ng/ml BPI | BPI | 10ng/ml Buffer Control |
| 10 | 333±18 | 601±257 | 270±23 | 270±67 | 436±38 | 697±37 |
| 100 | 769±101 | 1140±73 | 834±30 | 686±84 | 1005±50 | 892±47 |
| 1000 | 844±144 | 1016±20 | 1130±10 | 778±189 | 1025±71 | 723±88 |
| S. aureus | | | | | | |
| | 1685±121 | 1541±397 | 1558±139 | 1268±374 | 1554±324 | 1423±447 |

*BPI Protein or polymyxin B sulfate were preincubated with 0-10 ng/ml E. coli 0111:84 LPS or 0.1 % w/v killed S. aureus then added adherent peripheral blood mononuclear cells. TNF production was assayed by ELISA, and is reported as ng/ml.

## Example 5

### Inhibition of LPS-induced TNF production by neutrophil and recombinant CHO cell-derived BPI Protein

Human peripheral blood mononuclear cells were isolated on Ficoll-Paque (Pharmacia) gradients, washed 2X in pyrogen-free HBSS (Hazelton), and resuspended at $5 \times 10^6$/ml in RPMI (Gibco) media without serum. Two hundred μl of this cell suspension was incubated in each well of flat-bottom 96-well tissue culture dishes (Costar) for 2 hours at 37°C. Nonadherent cells were removed by washing 2X with RPMI + 10% autologous heat inactivated serum. Adherent mononuclear cells were stimulated with E. coli 0111:B4 LPS which had been preincubated for 30 minutes at 37°C with buffer, BPI Protein or polymyxin B (Gibco; 7900 U/ml). Supernatants were harvested four hours after LPS mixtures were added. Secretion of TNFα was quantitated by ELISA (Endogen) (results at Table 9).

Table 9

| Inhibition of LPS-Induced TNF Production by Human Monocytes | | | | | | | | | |
|---|---|---|---|---|---|---|---|---|---|
| | 10 ng/ml LPS | | | | 1 ng/ml LPS | | | | 0 LPS |
| BPI(nM): Lot No. | 0 | 7.3 | 1.4 | 0.3 | 0 | 7.3 | 1.4 | 0.3 | |
| 78038n | 626 | 129 | 159 | 203 | 334 | 153 | 187 | 165 | 113 |
| 148104n | | 98 | 104 | 162 | | 98 | 119 | 162 | |
| 148113n | | 92 | 114 | 151 | | 71 | 129 | 155 | |
| 148159r | | 82 | 158 | 155 | | 87 | 136 | 147 | |
| 148165r | | 124 | 128 | 138 | | 116 | 129 | 146 | |
| 148179r | | 85 | 139 | 134 | | 93 | 131 | 166 | |
| n=natural r=recombinant | | | | | | | | | |

Normal anesthetized mice were challenged by the intranasal route with 10 ng/ml E. coli 0111:B4 LPS (results in Table 10). Twenty minutes before challenge, anesthetized mice were treated by the intranasal route with 50μl saline, BPI Protein or polymyxin B solution. At one hour after LPS challenge, mice were re-anesthetized, and 0.7 ml of saline containing 1% human serum albumin was added to the lungs via the trachea. The lungs were gently kneaded. A 0.5 ml volume broncheoalveolar lavage (BAL) fluid was aspirated, cells were pelleted by centrifugation, and the BAL sample was stored at -70°C. The TNFα level in the BAL fluid was determined by measuring cytotoxicity towards WEHI clone 13 mouse fibrosarcoma cells and is summarized in Table 10. Human rTNFα (Chiron) was used as the standard.

Table 10

| Inhibition of TNFα Production as Measured in Broncheoalveolar Lavage Fluid | | | |
|---|---|---|---|
| Mouse | Saline Control | BPI 0.86μg (15 pmol) | Polymyxin B 1.0μg (782 pmol) |
| 1 | 1200 | 15 | 74 |
| 2 | 675 | 63 | 50 |
| 3 | 5560 | 425 | 132 |
| 4 | 2800 | 67 | 370 |
| 5 | 5250 | 1310 | 640 |
| Mean ± SD | 3097 ± 2250 | 376 ± 547 | 253 ± 251 |

## Example 6

### Expression of recombinant CHO cell-derived BPI Protein

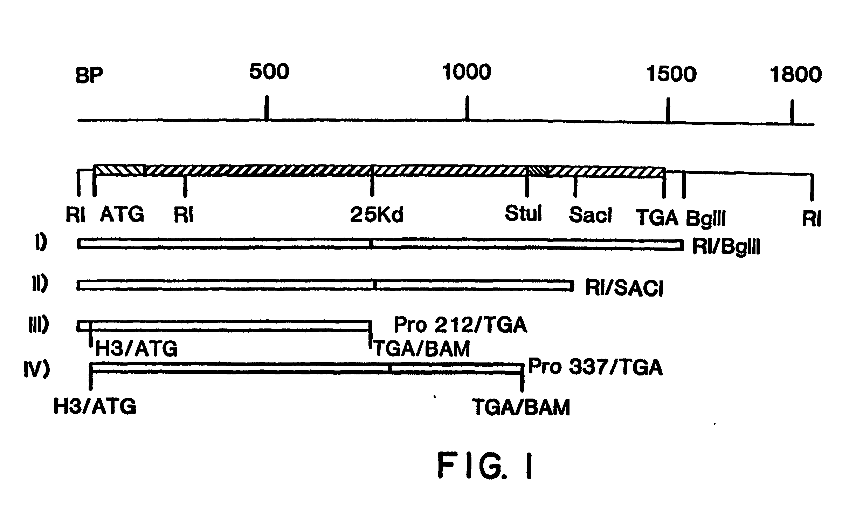

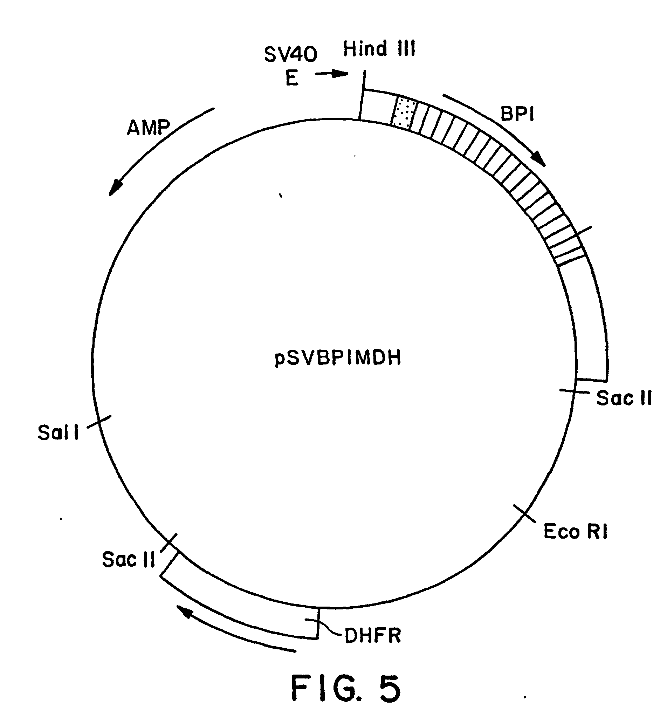

Recombinant CHO cell-derived BPI Protein was obtained as described in the following example. In order to produce BPI Protein and/or BPI Protein variants in mammalian cells, the cDNA sequences must be inserted into a suitable plasmid vector. A schematic drawing of cDNA encoding BPI Protein is shown in Figure 1. A suitable vector for such an application is pSV-1, which contains the origin of replication and early and late promoters of SV40, followed by multiple insert cloning sites, followed by the termination sequences from the hepatitis B surface antigen gene (Figure 5). Also contained within the plasmid are an origin of bacterial DNA replication, and the genes encoding ampicillin resistance and dihydrofolate reductase. Similar vectors have been used to express other foreign genes (McGrogan, et. al., Biotechnology 6, 172-177). Vector DNA was prepared for acceptance of BPI Protein cDNA sequences by digestion with HindIII and BamHI, and dephosphorylation with alkaline phosphatase.

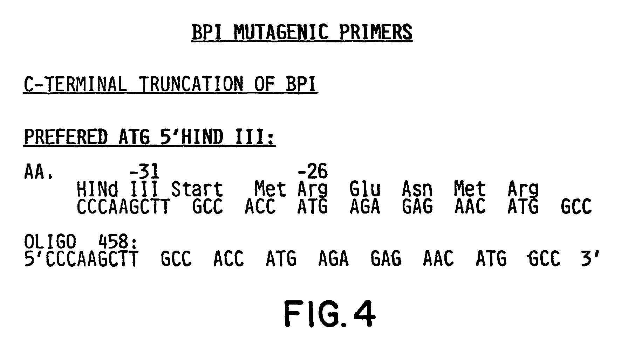

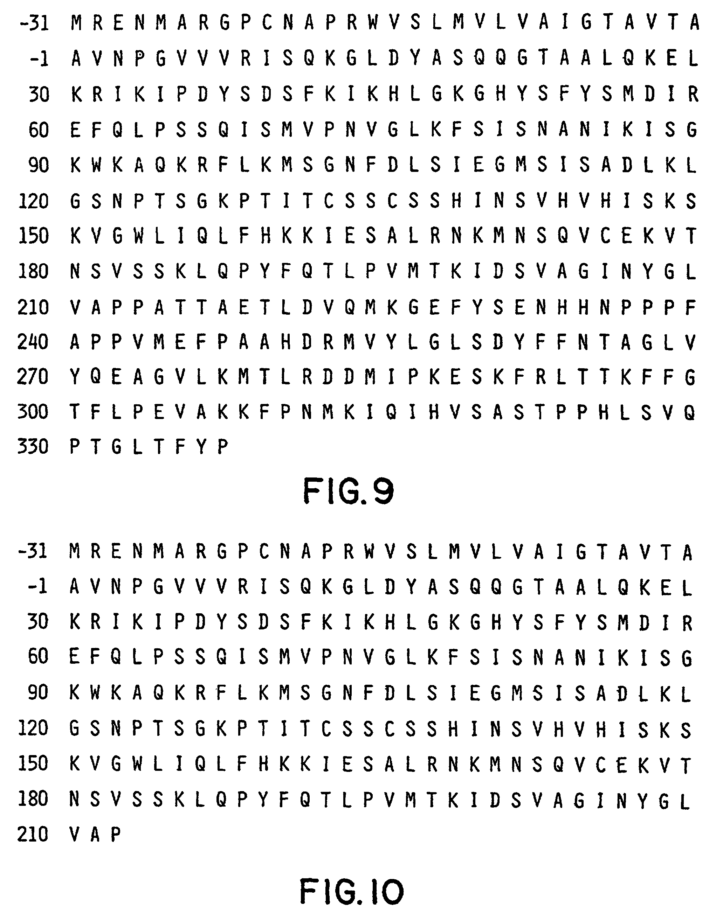

Several BPI Protein and BPI variant cDMA-containing inserts were prepared for insertion into pSV-1 First, an insert encoding BPI Protein was prepared by digestion of the parent plasmid with appropriate restriction enzymes, for example, EcoRI and BglII, yielding two DNA fragments containing portions of the BPI Protein coding sequence. These two fragments were ligated together into prepared SV-1, and the recombinant clones obtained were screened by restriction enzyme digestion for the presence of the two inserts in the proper orientation. Two cDNAs encoding truncated forms of BPI Protein were generated using oligonucleotide-directed DNA amplification of the parent BPI Protein insert DNA. The amplifying oligos were designed to replace codons 212 (oligo 459) (Figure 2) and 337 (oligo 460) (Figure 3) with stop codons, in addition to a BamHI cloning site. At the 5'-end of both constructs, oligo 458 was used in the amplifications to create a HindIII site immediately upstream of the translational start codon ATG (Figure 4). Thus, three BPI-variant encoding inserts were created, each encoding 55 kDa, 38 kDa, and 25 kDa portions of BPI Protein, and each was ligated separately into prepared vector DNA. Figures 9 and 10 show the amino acid sequences of the 38 kDa and 25 kDa portions, respectively.

Each of the three constructs was verified by restriction digest analysis, and then prepared in amounts sufficient for transfection into CHO cell line DUXB11 cells. Transfection was performed using lipofectin, and the resulting transformed cells were selected in the presence of increasing amounts of methotrexate using protocols which are standard in the art. The transformed cells were cultured under conditions standard in the art, and supernatants from either transfected pools or clones derived from the pools were assayed for BPI Protein or BPI variant by the BPI Protein ELISA method discussed infra.

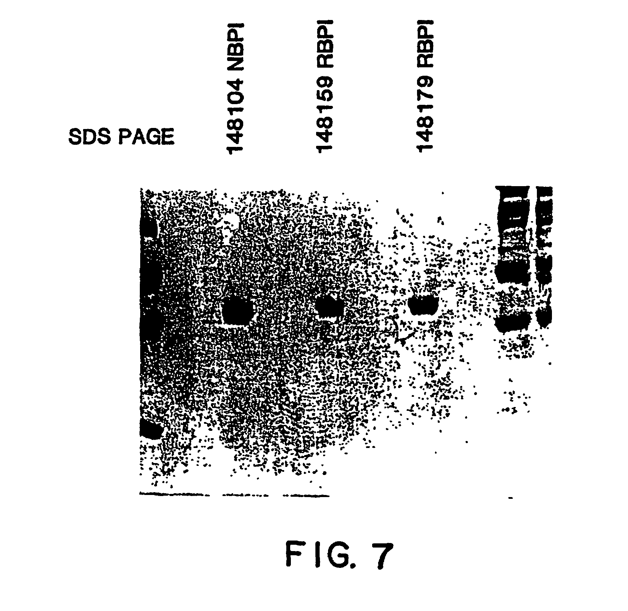

Figure 7 shows SDS-PAGE analysis of native BPI Protein, and two lots of CHO-derived recombinant BPI Protein. Recombinant BPI Protein migrates as a single band on an SDS-PAGE gel at about 55 kDa. Recombinant BPI Proteins produced in CHO cells exhibit a slightly altered migration pattern on SDS-PAGE from native protein, indicating that the molecule may also be processed differently in CHO cells than in neutrophils or HL60 cells. Such processing may be either responsible for, or a result of, the molecule being secreted rather than packaged into granule membranes.

Amino acid analyses of recombinant BPI and native BPI are shown in Table 11 which shows the two to be essentially the same.

Table 11

| Amino Acid Composition of recombinant BPI 148179 | | |
|---|---|---|
| | recombinant | native |
| D+N | 27 | 38 |
| E+Q | 35 | 39 |
| S | 47 | 44 |
| G | 31 | 26 |
| H | 12 | 14 |
| R | 17 | 12 |
| T | 21 | 21 |
| A | 26 | 25 |
| P | 33 | 32 |
| Y | 16 | 14 |
| V | 33 | 37 |
| M | 10 | 15 |
| I | 26 | 26 |
| L | 50 | 47 |
| F | 25 | 25 |
| K | 42 | 36 |
| total | 451 | 451 |
| (C,W not quantitated) | | |

## Example 7

### Expression of recombinant BPI Protein in insect cells

1. construction of plasmid expression vector

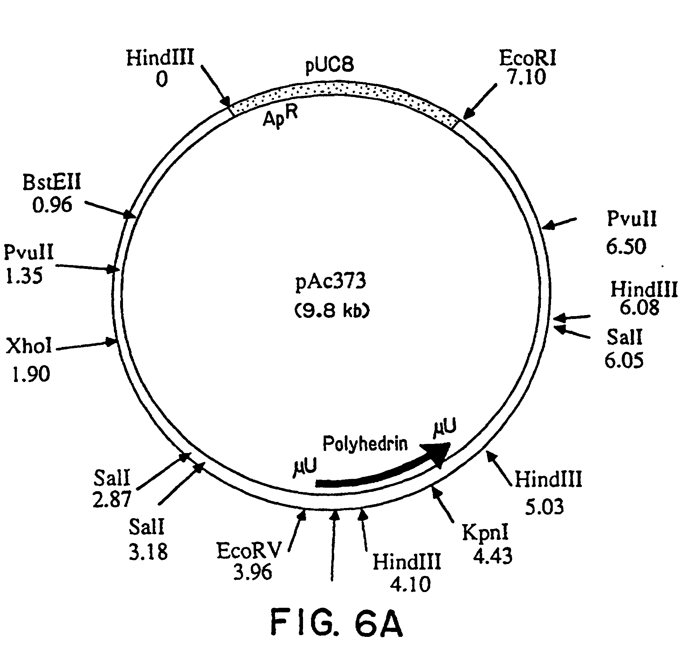

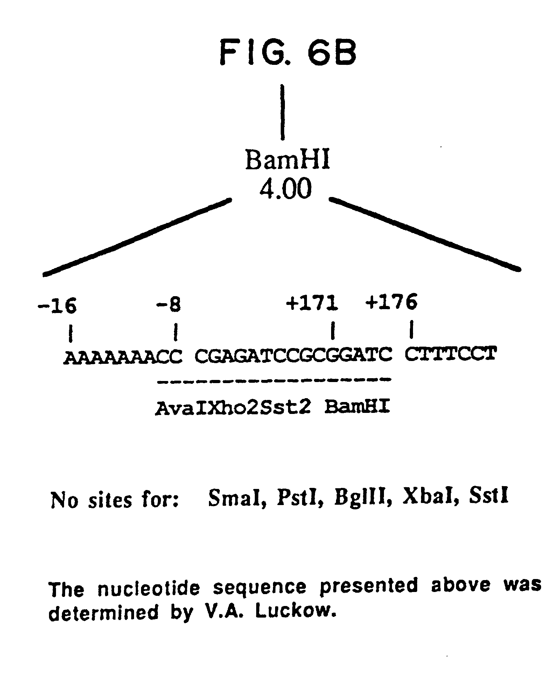

In order to produce BPI Protein and/or BPI Protein variants in insect cells, the cDMA sequence must first be inserted into a suitable plasmid expression vector, such as pAC373 (see Figures 6-1 and 6-2). Appropriate restriction sites for this insertion were created by standard site-directed mutagenesis procedures. The essential properties of a suitable expression vector include a transcriptional promoter such as the polyhedron gene promoter of pAC373, and flanking homologous sequences to direct recombination into the baculovirus genome. A polyadenylation signal, such as the one from the polyhedron gene present in this plasmid vector, may or may not be necessary for expression of the recombinant gene. A marker gene such as the beta-galactosidase gene of E. coli, juxtaposed to regulatory sequences including a transcriptional promoter and possibly a polyadenylation signal, may be included in the vector but is not essential for Bactericidal/Permeability Increasing Protein expression. A typical vector for such purposes, pAC373, is shown in Figures 6-1 and 6-2.

2. Creation of recombinant baculovirus

A chimeric baculovirus was created by homologous recombination between the expression plasmid containing the BPI Protein target gene (or truncations thereof derived as described supra) and wild type baculovirus DNA. Plasmid and wild type baculovirus DNA were co-precipitated by the calcium phosphate technique and added to uninfected Spodoptera frugiperda (Sf9) insect cells. Four to seven days following transfection, cells exhibited a cytopathic morphology and contained the nuclear occlusion bodies typically produced by viral infection. The cell-free culture media containing both wild type and recombinant virus was harvested and assayed for BPI Protein activity.

3. Identification and isolation of chimeric baculovirus

Clonal isolates of virus was obtained from this co-transfection stock by plaque purification on Sf9 cell monolayers overlaid with agarose. Candidate plaques for analysis will be identified by a plaque morphology negative for occlusion bodies. If the expression plasmid contains a marker gene such as beta galactosidase, recombinant plaques will be indicated by the blue color produced from a chromogenic substrate such as 5-bromo-4-chloryl-3-indolyl-β-D-galactopyranoside (X-gal) in the agarose plating medium. Picked plaques will be used for inoculation of cells in multi-well dishes. The resulting cell lysates and infected cell supernatants can be evaluated for expression of recombinant BPI Protein using standard activity or immunological assays. Positive wells may require additional rounds of plaque purification to obtain pure recombinant virus stocks free from wild type contamination.

4. Batch production of BPI Protein

Sf9 cells were adapted to growth in serum-free, low protein medium such as ExCell (J.R. Scientific). Cells were collected from suspension culture by gentle centrifugation and resuspended in fresh medium containing the viral inoculum at a concentration of ten million cells per ml, using a multiplicity of infection of one virus plaque-forming unit per cell. After a period of two hours, the culture was diluted five-fold with fresh medium and incubated for two to three days. At the end of that time, the cells were pelleted by centrifugation and the conditioned medium was harvested. BPI Protein was purified from the cell-free supernatant by standard means.

5. Characterization of insect cell derived BPI Protein

BPI Protein produced in insect cells using a baculovirus expression system is a glycosylated protein of approximate molecular weight of 55,000 kd. The N-terminal amino acid sequence is identical to that of mature mammalian cell-produced BPI Protein, indicating correct processing of the signal sequence. The specific activity of endotoxin binding of recombinant protein was indistinguishable from BPI Protein obtained from natural sources.

**Example 8**

**Expression of recombinant BPI Protein with signal sequence in E. Coli**

A. Construction of pT7BPI protein plasmids

Oligonucleotides were prepared on an Applied Biosystems 380B DNA synthesizer for use in oligonucleotide-directed DNA amplification. The oligonucleotides were created with NdeII and BamHI restriction sites at the 5' and 3' ends, respectively, of the BPI Protein DNA. In addition, another oligonucleotide containing a BamHI restriction site was used to create the truncated proline-212 version of the BPI Protein DNA.

Following the amplification reactions, fragments were purified and digested with NdeI and BamHI. The plasmid pGEMEX-1 (available from Promega) was selected as the vector for the constructions. pGEMEX-1 contains a T7 promoter which can be used for the expression of downstream sequences when placed into the proper host. The vector was cleaved with BamHI and, following purification, partially digested with NdeI to generate a vector with a single NdeI site and a single BamHI site. The fragments were ligated and transformed into the E. coli strain JM101 using the Hanahan transformation protocol. The transformed bacteria were plated on LB plates containing carbamicillin and incubated overnight at 37°C. Resistant colonies were selected and analyzed by preparing mini-plasmid preparations and digesting with the appropriate restriction enzymes. Digests were analyzed on both 1% agarose gels and 5% polyacrylamide gels.

The expression host, E. coli strain JM109(DE3), was transformed using 1 µl of the mini-plasmid preparation and the Hanahan transformation protocol (DNA Cloning: A Practical Approach, Vol I, D.M. Glover Ed., IRC Pr. Ltd., Chapter 6, pp. 109-135 (1985)). JM109(DE3) contains a chromosomal copy of the gene for T7 RNA polymerase which can be induced with IPTG. The transformed bacteria were plated on LB plates containing carbamicillin and incubated overnight at 37°C. Results are not shown.

B. Results

When a signal sequence is operably linked to the mature BPI Protein cDNA sequence in the expression plasmid as provided in a full length clone and reported by Gray, et al. [Journ. of Biol. Chem., 264:9505 (1989)], no bacterial colonies are obtained, whereas numerous colonies can be obtained if the signal sequence is deleted. Since the full-length and proline-212 truncated forms of BPI Protein containing the signal peptide do not give colonies while those forms that do not contain the signal peptide do give colonies, the BPI Protein was expressed in an active form and is processed correctly, sending the protein to the periplasmic space of the bacteria (the location in bacteria to which proteins possessing a signal peptide are sent) where the bactericidal activity kills the cell. This also implies that both the full-length form and the proline-212 truncated form are active and capable of bactericidal activity.

**Example 9**

**Expression of recombinant E. Coli-derived BPI Protein**

1. Generation of BPI Protein expression vector

To generate a construct for E. coli expression, the BPI Protein encoding sequence was subcloned from the CHO cell expression vector by PCR using the oligonucleotides: BPI.forward 5'C.ACC.GCC.GTG.ACA.CAT.ATG.GTC. AAC.CCT.GGC.GTC BPI.reverse 5'GAC.GTT.CTC.TAT.AAA. TGA.AGG.ATC.CTA.GCT.C generating a NdeI and Bam-HI site respectively. The resulting fragment was cut with NdeI and BamHI and subcloned into pGEMEX-1 VECTOR (Promega).

The pGEMEX E. coli expression vector contains three RNA polymerase fragments. Downstream from gene 10 is a multiple cloning sequence. In-frame subcloning can be performed to generate fusion proteins, if the protein of interest has the appropriate restriction sites in-frame.

To avoid the problems associated with fusion protein production, the gene 10 region was removed from pGEMEX, but the T7 RNA polymerase binding site and the NdeI and BamHI cloning sites were retained. To accomplish this the NdeI site at 3251 was removed by partial digest followed by Klenow fill-in and re-ligation. This plasmid is referred to as pT7BPI. The correct sequence was confirmed by sequencing the entire coding region for BPI Protein but with the leader sequence deleted, such that the first amino acid coded for by the plasmid is methionine.

2. Mutagenesis for Construction of BPI variants

The plasmid pT7BPI has an f1 ori for the production of single-stranded DNA. This pT7BPI was transformed into the E. coli strain CJ236 for the production of ssDNA to be used as a template for site-directed mutagenesis according to the method of Kunkel [(Kunkel, T.A. (1988) in Nucleic Acids and Molecular Biology (Eckstein, F., Lilley, D. M.J. Eds.) Vol.2, p. 124, Springer-Verlag, Berlin and Heidelberg). Mutagenic oligonucleotides were generated with homology on the 5' side and 12 nucleotides of exact homology on the 3' side of the mismatched codon or codons corresponding to the site of mutation. The mutagenic oligonucleotide was purified using OPC columns as described by ABI, phosphorylated by standard enzymatic techniques, annealed to ss-pT7BPI generated in CJ236 cells, extended at 37°C with T7DNA polymerase and ligated with T4 ligase. The resultant closed circular ds-DNA was transformed according to standard protocols (see, e.g., Sambrook, et al.) into RecA+ E. coli such as JM101 and plated. Single colonies were grown for production on ds-DNA by standard techniques for double-stranded sequencing. Clones with the correct mutation were transformed into JM109 (DE3) for expression.

3. Expression of BPI Protein

JM109 (DE3) contains a chromosomal copy of the gene which codes for T7RNA polymerase under the control of the inducible lac promoter. JM109 (DE3) containing pTBPI (or a variant of BPI) is grown overnight in 2xYT media (Bio101, La Jolla, CA) + 0.2% glucose + 100mg/ml carbenicillin at 26-37°C. Low temperature and high nutrient-containing solution is helpful in generating productive inoculants. The inoculum is diluted 1:250 to 1:500 and grown to $OD_{600nm} \cong 1$ in a shake flask or $OD_{600nm} \cong SO$ in a fermentor at 26-37°C, and induced with IPTG at 0.1-1.0 mM for 4-24 hours. The bacteria are collected by centrifugation, resuspended in 10 mM TRIS, pH 8, 1 mM EDTA, and disrupted by high pressure homogenization. When bacteria were grown at 37°C as is standard in the art, essentially all of the BPI Protein was produced insoluble in inclusion bodies. However, when the temperature was lowered below 37°C, increasing amounts of BPI Protein were recovered in soluble, active form.

**Results**

1. Expression of insoluble BPI Protein in E. coli

Expression at 37°C resulted predominantly in the production of BPI Protein in insoluble inclusion bodies, as identified by light microscopy. E. coli cells were lysed in Tris pH 6.0 with an Ultra-Turrax homogenizer. Inclusion bodies were collected by centrifugation, washed with 1 M NaCl 0.05% triethylamine, and the protein refolded by rapid dilution from a 6M guanidine solution into various buffers. BPI Protein was purified by a capture of Fast S Sepharose and eluted with 0.6 M NaCl, diluted to 0.25 M NaCl and passed over Fast Q Sepharose to remove endotoxin and recaptured on Fast S Sepharose and eluted by 0.6 M NaCl or a gradient of 0.25 to 1 M NaCl. Refolding under a variety of conditions resulted in active BPI Protein with approximately 0.1% yield from all BPI (i.e., 1mg/g total BPI).

## 2. Expression of soluble active BPI Protein in E. coli

Alternatively, it was discovered that BPI Protein can be generated in a soluble form within E. coli by adjusting the fermentation conditions. A greater yield of soluble BPI Protein was discovered as the fermentation temperature is decreased from 37°C to 26°C with a concomitant loss in inclusion body material. This was demonstrated in shake flask cultures grown in 2xYT media (Bio101, La Jolla, CA) + 0.2% glucose + 100mg/ml carbenicillin. The ability to produce BPI Protein is quite unpredicted, since BPI Protein is bactericidal for E. coli and BPI Protein constructs with a leader sequence designed to secrete BPI Protein into periplasmic space resulted in cell death. However, it is shown here that BPI Protein not secreted but retained in the cytoplasm can be maintained in high concentrations without cell death. To purify soluble BPI Protein, the cell lysate from the disruption step (disruption by microfluidizer) was clarified by centrifugation and filtration. followed by centrifugation and filtration, and the soluble protein purified as described below. Alternatively, cell lysate can be clarified by treatment with polycations such as polyethyleneimine or Biocryl (Toso Haas). The generation of soluble material has many advantages, i.e., there are no refolding steps and there is greater reproducibility from batch to batch.

BPI Protein was produced with yields corresponding to about 1 mg per liter of production at a cell density of 1 $OD_{600nm}$. By using standard fed-batch fermentation techniques, the overall production can be enhanced significantly to achieve cell densities of greater than 50 $OD_{600nm}$. One example would be to inoculate the bacteria in a media consisting of 8 g per liter tryptone, 5 g per liter yeast extract, 2.5 g per liter NaCl, 2 g per liter glucose, and 50 microgram per ml carbenicillin, with a feed of 100 g per liter tryptone, 60g per liter yeast extract, 200 g per liter glucose and 0.1 g per liter carbenicillin and a maintained pH of 7.1 and dissolved oxygen at 50%. This E. coli synthesis of a protein toxic to E. coli represents a substantial advance in the state of the art of BPI Protein production.

Fermentation conditions can be varied by those skilled in the art to maximize production. Further advances to increase the amount of soluble BPI Protein include overexpression of cellular cofactors or chaperon proteins that are necessary or advantageous to BPI Protein folding in the cell. Also, one could optimize the amino acid structure of BPI Protein, while maintaining its biological activity, so as to produce an active BPI Protein variant which is expressed at a high level in bacteria.

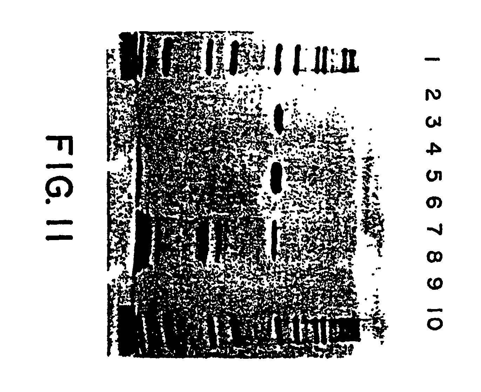

BPI Protein produced in Chinese hamster ovary cells migrate with a higher molecular mass on sodium dodecyl sulfate polyacrylamide gel electrophoresis (SDS-PAGE) than E. coli-produced BPI Protein, indicating that these molecules are processed differently in mammalian cells than in E. coli (Figure 11).

## 3. Purification of E coli-derived BPI Protein

The purification of BPI Protein from E. coli lysates presents several obstacles to be overcome. BPI Protein is a potent endotoxin-binding molecule, and experimental evidence has shown there to be more than 100 million endotoxin units per milliliter in the E. coli cell lysate (based on the limulus amebocyte lysate assay). Thus the BPI Protein produced in E. coli is complexed to endotoxin and associated cell wall fragments. To obtain purified soluble active BPI Protein from E. coli, the BPI Protein must be purified away from its endotoxin while retaining its activity. These difficulties have now been overcome with a combination of steps designed to produce highly purified, soluble, active, endotoxin-free BPI Protein from E. coli.

## 4. Purification Process A

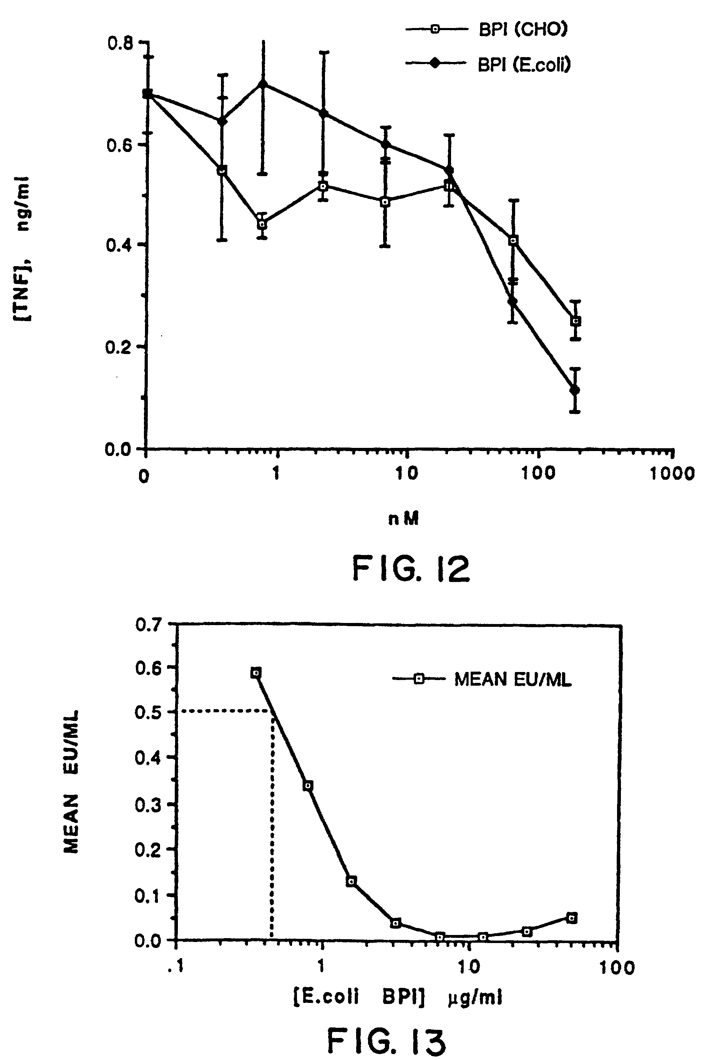

E. coli bacteria expressing BPI Protein were lysed by high pressure homogenization (Microfluidics, Newton, MA) in 10mM Tris 1mM EDTA pH 8. Centrifugation and filtration were used to remove remaining whole cells and cell debris. $MgCl_2$ was added to 500mM, and incubated for 30 minutes at 37°C. Acetic acid was added to lower pH to 4.0. Additional incubation, 30 minutes at 37°C, caused precipitation of much of the contaminating protein, lipid, and DNA. After centrifugation and filtration, BPI Protein was left soluble in the supernatant. It was diluted 3x and bound to a cation exchanger, e.g., Poros HS (Perseptive Biosystems, Cambridge, MA), Fast S Sepharose, or CM Sepharose (Pharmacia, Piscataway, NJ). A series of washes followed: pH 6.5, 8.5 and 10.0. The pH was brought back to 6 using a suitable buffer, and elution was achieved with a 0-2M NaCl gradient or step elution. The pH of the eluant was raised to 8.5, and the solution was passed through a suitable anion exchanger, e.g. Poros HQ, Fast Q Sepharose, or DEAE Sepharose, to remove any remaining endotoxin and DNA, as well as contaminating anionic compounds. BPI Protein does not bind under these conditions. BPI Protein was then bound to another cation exchange column, and eluted with either a gradient or a step of NaCl. The N-terminal sequence of BPI Protein was confirmed by automated Edman degradation and activity verified by inhibition in the Limulus amebocyte lysate assay (Figure 13).

### 5. Purification Process B

BPI Protein complexed with endotoxin, as it is in the cell lysate, will not bind to cation exchange. Thus, as an alternative purification scheme, the cell lysate can be passed over a cation exchange column, such as Poros HS (Perseptive Biosystems Cambridge, MA), Fast S Sepharose, or CM Sepharose (Pharmacia, Piscataway, NJ), at the lowest possible pH at which the BPI Protein-endotoxin complex will not bind. This removes many of the contaminating cationic entities which would otherwise co-elute with BPI Protein in a subsequent cation exchange step. The column flow through is then brought to 500mM $MgCl_2$, incubated 30 minutes at 37°C and pH adjusted to 4.0 with acetic acid. After centrifugation and filtration, BPI Protein is soluble in the supernatant. BPI Protein is then diluted 3x and bound to a cation exchanger, e.g., Poros HS (Perseptive Biosystems, Cambridge, MA), Fast S Sepharose, or CM Sepharose (Pharmacia, Piscataway, NJ). A series of washes would follow: pH 6.5, 8.5 and 10.0. The pH is then brought back to 6 in a suitable buffer, and elution achieved with a 0-2M NaCl gradient or step elution. The pH of the eluant is then raised to 8.5, and the solution passed through a suitable anion exchanger, e.g., Poros HQ, Fast Q Sepharose, or DEAE Sepharose to remove any remaining endotoxin and DNA, as well as contaminating anionic compounds. BPI Protein does not bind under these conditions. BPI Protein is then bound to another cation exchange column, and eluted with either a gradient or a step of NaCl. BPI Protein is then formulated in 10mM Sodium Succinate, 110mM NaCl, pH 6.0 by size exclusion chromatography using Sepharose CL-6B.

### 6. Purification Process C

An alternative lysis protocol would involve using additives that would prevent the formation of BPI Protein-endotoxin complexes. Magnesium Chloride would be added to the cell suspension prior to lysis, with pH at either 8 or 4. Lysis would be achieved by high pressure homogenization. After centrifugation and filtration, BPI Protein would be soluble in the supernatant. BPI Protein would be diluted 3x and bound to a cation exchanger, e.g., Poros HS (Perseptive Biosystems, Cambridge, MA), Fast S Sepharose, or CM Sepharose(Pharmacia, Piscataway, NJ). A series of washes would follow: pH 6.5, 8.5, 10.0. The pH would be brought back to 6 in a suitable buffer, and elution achieved with a 0-2M NaCl gradient or step elution. The pH of the eluant would be raised to 8.5, and the solution passed through a suitable anion exchanger, e.g. Poros HQ, Fast Q Sepharose, or DEAE Sepharose to remove any remaining endotoxin and DNA, as well as contaminating anionic compounds. BPI Protein does not bind under these conditions. BPI Protein would then be bound to another cation exchange column, and eluted with either a gradient or a step of NaCl. BPI Protein would then be formulated in 10mM Sodium Succinate, 110mM NaCl, pH 6.0 by size exclusion chromatography using Sepharose CL-6B.

### 7. Use of endotoxin removal agents

Endotoxin-removing polycations such as polyethylimine or BioCryl (Toso Haas) can be added to cells which are next lysed by high pressure homogenization. After centrifugation and filtration, BPI Protein would be soluble in the supernatant. BPI Protein would be bound to a cation exchanger, e.g. Poros HS (Perseptive Biosystems, Cambridge, MA), Fast S Sepharose, or CM Sepharose (Pharmacia, Piscataway, NJ). A series of washes would follow: pH 6.5, 8.2 and 10.0. The pH would be brought back to 6 in a suitable buffer, and elution achieved with a 0-2M NaCl gradient or step elution. The pH of the eluant would be raised to 8.5, and the solution passed through a suitable anion exchanger, e.g., Poros HQ, Fast Q Sepharose, or DEAE Sepharose to remove any remaining endotoxin and DNA, as well as contaminating anionic compounds. BPI Protein does not bind under these conditions. BPI Protein would then be bound to another cation exchange column, and eluted with either a gradient or a step of NaCl. BPI Protein would then be formulated in 10mM Sodium Succinate 110mM NaCl pH 6.0 by size exclusion chromatography using Sepharose CL-6B.

### 8. Removal of endotoxin from the outer membrane prior to cell lysis

Alternatively, the outer membrane could be removed from the bacteria prior to lysis, thus removing the endotoxin and the need for subsequent endotoxin removal in the purification. An example would be to add bivalent chelating reagents such as EDTA or EGTA or by adding a detergent such as Triton X-100 [S.T.L. Harrison, Biotech. Adv. 9: 217-240 (1991)].

### 9. Removal of endotoxin from BPI Protein by chromatographic processes

Endotoxin can be removed from BPI Protein by passing BPI Protein through an anion exchanger, e.g., Poros HQ, Fast Q Sepharose, or DEAE Sepharose, at a high pH (8.5 or above), or by binding BPI Protein to a cation exchanger, e.g., Poros HS (Perseptive Biosystems, Cambridge, MA), Fast S Sepharose, or CM Sepharose (Pharmacia, Piscataway, NJ), and washing extensively at high pH prior to elution with NaCl. The BPI Protein-expressing E. coli could be suspended in low pH buffer, e.g., 20mM Sodium Acetate pH 4.0, prior to lysis by high pressure homogenization. Alternatively, the pH could be lowered after lysis. After centrifugation and filtration, BPI Protein would be soluble in the supernatant. BPI Protein would be bound to a cation exchanger, e.g., Poros HS (Perseptive Biosystems, Cambridge, MA), Fast S Sepharose, or CM Sepharose (Pharmacia, Piscataway, NJ). A series of washes would follow: pH 6.5, 8.5 and 10.0. The pH would be brought back to 6 in a suitable buffer, and elution achieved with a 0-2M NaCl gradient or step elution. The pH of the eluant would be raised to 8.5, and the solution passed through a suitable anion exchanger, e.g., Poros HQ, Fast Q Sepharose, or DEAE Sepharose to remove any remaining endotoxin and DNA, as well as contaminating anionic compounds. BPI Protein does not bind under these conditions. BPI Protein would then be bound to another cation exchange column, and eluted with either a gradient or step of NaCl. BPI Protein would then be formulated in 10mM Sodium Succinate, 110mM NaCl, pH 6.0 by size exclusion chromatography using Sepharose CL-6B.

### 10. Activity Assay of BPI Protein

BPI Protein produced in soluble form at low temperature in E. coli and purified as described above in purification process A was tested for ability to neutralize endotoxin in the limulus amebocyte lysate (LAL) assay. This assay is based on the ability of gram-negative bacterial endotoxin to catalyze the activation of a pro-enzyme in the LAL. The initial rate of activation is determined by the rate of color formation using the substrate Ac-Ile-Glu-Gly-Arg-pNA and monitoring the absorbance at 405nm photometrically after the reaction is stopped. There is a linear correlation between absorbance at 405 nm and endotoxin concentration between 0.1 and 1 EU/ml.

Accordingly, 1EU of endotoxin was mixed with a dilution series of E. coli BPI Protein in a microtiter plate at 37°C followed by substrate addition. The absorbance in each well of the plate was measured using a Molecular Devices plate reader. Figure 13 shows the inhibition of LAL activation by E. coli BPI Protein, with an $IC_{50}$ = 0.45 mg/ml. Thus, E. coli-derived BPI Protein is able to bind and neutralize endotoxin.

BPI Protein produced in soluble form at low temperature in E. coli and purified as described above in purification process A was tested for inhibition of endotoxin-mediated TNF induction by human adherent mononuclear cells. Blood collected in acid citrate dextrose containing vacutainer tubes (Becton Dickinson, Rutherford, NJ) was diluted in Hank's balanced salt solution (HBSS) minus $Ca^{+2}$ and $Mg^{+2}$. Mononuclear cells were separated using Ficol-Paque (Pharmacia Inc., Piscataway, N.J.), collected and washed three times in HBSS, and the proportion of monocytes was estimated by microscopic examination. Cells were brought up to an appropriate volume of RPMI 1640 with glutamine and antibiotics and without serum to give approximately $1 \times 10^6$ monocytes/ml. Cells were plated into 96 11 flat bottom tissue culture plates (Costar, Cambridge, MA), 200 μl/well, and incubated for two hours at 37°C in a humidified incubator with 7% $O_2$. Cells were then washed three times in warm RPMI 1640 without serum. After the last wash was aspirated, 200 μl/well RPMI 1640 with 10% autologous heat-inactivated serum was added. To each well was then added the 22μl of 10X solution of E. coli endotoxin preincubated in buffer, polymyxin B, or BPI Protein. Cells were incubated with the endotoxin mixture for four hours at 37°C, then the supernatants were collected and assayed for TNFα antigen by ELISA (Endogen, Inc., Boston, MA) (Figure 12) .

### 11. BPI Protein ELISA

#### A. Materials

Immulon-4 96 well plates (Dynatech Labs, 011-010-3750); 12-channel 50-200μl pipettor; P20, P200, P1000 pipettors; micropipette and multichannel pipette tips; reagent reservoirs (Costar); racked 1 ml tubes (BioRad); and polypropylene 15 ml conical tubes.

#### B. Reagents

Coating Buffer: 25 mM Sodium Borate pH 9.5. Blocking Buffer: 5% BSA (Sigma A-7030) in PBS. ELISA Buffer: 50 mM Tris pH 7.4, 150 mM NaCl, 1 mg/ml BSA (Sigma A-7030), 0.05% Tween 20 (Sigma, P-1379), 1 μg/ml Polymyxin B Sulfate (Gibco/BRL, 7900 U/mg).

Standard and sample diluent = Wash/Sample Buffer with 60 mM $MgCl_2$ or appropriate solution for unknowns (e.g. if testing tissue culture supernatants, use REM + dFBS).

Substrate Buffer: 24.5 mg $MgCl_2$ (make 500 ml), 48 ml Diethanolamine, bring up to ~ 400 ml with Lab V $H_2O$, adjust to pH 9.8, bring up to 500 ml with Lab V $H_2O$.

Antibodies: INV 1-2 IgG (protein A purified Rb anti BPI antibody, 5 mg/ml); and biotinylated anti-BPI antibody. Also, monoclonal antibody HA-1A may be used. Anti-BPI antibodies may be produced, for example, by immunizing a rabbit with BPI, and recovering the anti-BPI antibodies produced thereby using methods known to those skilled in the art.

Other Reagents: BPI standard (Lot 149713 = 1.0 mg/ml aliquots stored at -70°C); PNPP substrate tablets (5 mg/tablet: Sigma 104-105); Streptavidin-Alkaline Phosphatase (BioRad, 170-3554, Lot 72439). BPI may be obtained, for example, according to the method described at column 11, line 62 through column 12, line 13 of U.S. Patent No. 5,089,274.

C. Procedure

Coating Plates: Note: Coat plates up to 1 month in advance. Store plates at 4°C until needed. Dilute anti-BPI IgG antibody (protein A-purified) to 5 µg/ml in 25mM Na Borate, pH 9.5 (10 ml/plate). Add 100 µl to each well of 96-well plate (Immulon-4). Incubate overnight at 37°C. Refrigerate until used.

Blocking Plates: Flick coating antibody out of plates and blot plate on paper towels. Add 200 µl 5% BSA in PBS to each well. Incubate 1-4 hours at 37°C or overnight at 4°C. Flick out blocking solution, wash 3X with ELISA buffer and blot plate on paper towels.

BPI Standards and Unknowns: Note - BPI standards and samples should be diluted with ELISA buffer/60mM MgCl$_2$ and only in propylene tubes! Thaw new standard aliquot (0.5 ml at 1 mg/ml) every 2 months. Make stock solution of purified BPI at 100 ng/ml by: a) diluting 149713 BPI 5 µl + 495 µl diluent (= 1:100; 10 µg/ml), then again 1:100 for a final concentration of 100 ng/ml in 1 ml (this is enough 100 ng/ml stock solution for 2 standard curves in triplicate); and b) making 1000 µl of each of the following standard concentrations as follows:

| Final [BPI] ng/ml | 100 ng/ml BPI (µl) | buffer (µl) |
| --- | --- | --- |
| 15 | 150 | 850 |
| 5 | 50 | 950 |
| 1 | 20 | 1980 |
| 0.50 | 0 | 500 + 500 of 1ng/ml |
| 0.25 | 0 | 750 + 250 of 1ng/ml |
| 0.10 | 0 | 900 + 100 of 1ng/ml |
| 0.05 | 0 | 9500 + 50 of 1ng/ml |
| 0 | 0 | 1000 |

Add 100 µl/well standards and unknowns and incubate at 37°C for 2 hours. Wash 4 X with Sample/Wash Buffer and vigorously blot on paper towels.

2nd Antibody (Conjugate): After final wash, blot plate vigorously, add 100 µl of biotinylated anti-BPI Ab at 1:2000 (=5µl in 10ml of wash/sample buffer) to each well. Incubate at 37°C for 1 hour, or overnight at 4°C. Wash 4 X and blot dry.

Streptavidin Alkaline Phosphatase: After final wash, blot plate vigorously, add 100 µl 1:1000 (=10µl in 10ml) Steptavidin-Alkaline Phosphatase conjugate to each well. Incubate at 37°C for 45 minutes, or overnight at 4°C. Wash 4 X and blot dry.

Substrate: After final wash, blot plate vigorously, and add 100 µl substrate solution (2 X 5 mg PNPP substrate tablet/10 ml substrate buffer). Note: Prepare fresh substrate immediately before use by dissolving 2 tablets/10ml substrate buffer.

Read plate at 405nm after developing for 25-35 min. Keep plate in the dark between readings.

**Claims**

1. A method of producing non-glycosylated Bactericidal/Permeability Increasing Protein using a gram-negative bacterial cell, the method comprising:

   transforming or transfecting the cell with a vector encoding Bactericidal/Permeability Increasing Protein, wherein the Bactericidal/Permeability Increasing Protein has no leader peptide attached thereto when expressed;
   culturing the cell so transformed or transfected so as to produce non-glycosylated, Bactericidal/Permeability Increasing Protein in the cell; and
   recovering from the cell the non-glycosylated Bactericidal/Permeability Increasing Protein so produced;

**characterised in that** the cell is cultured at a temperature below 37°C.

2.  A method according to Claim 1, wherein the cell is cultured at a temperature between 25-36°C.

3.  A method according to Claim 2, wherein the cell is cultured at a temperature of 26°C.

4.  A method according to any preceding claim wherein the recovering comprises:

    (a) lysing the cell under conditions preventing the formation of a complex between the Bactericidal/Permeability Increasing Protein and LPS so as to release the Bactericidal/Permeability Increasing Protein from the cell; and
    (b) isolating the Bactericidal/Permeability Increasing Protein so released.

5.  A method according to any one of Claims 1 to 3 wherein the recovering comprises:

    (a) lysing the cell under conditions permitting the formation of a complex between the Bactericidal/Permeability Increasing Protein and LPS ;
    (b) isolating the resulting Bactericidal/Permeability Increasing Protein-LPS complex;
    (c) dissociating the Bactericidal/Permeability Increasing Protein-LPS complex so isolated, so as to release the Bactericidal/Permeability Increasing Protein from the Bactericidal/Permeability Increasing Protein-LPS complex; and
    (d) isolating the Bactericidal/Permeability Increasing Protein so released.

6.  A method according to any preceding claim wherein the Bactericidal/Permeability Increasing Protein is the native 55 kD human protein having the amino acid sequence shown for human BPI Protein in Figures 8A-1 and 8A-2, and encoded by the cDNA sequence shown in Figures 8B-1 and 8B-2, or a BPI variant which comprises a portion of BPI protein, which variant is capable of (a) binding to LPS, (b) competing with BPI Protein or LBP for binding to LPS, and (c) inhibiting the LPS-mediated production of TNF$\alpha$ by human monocytes.

7.  A method according to Claim 6, wherein the BPI variant is $B_{(S351->A)}$, $B_{(D200->DP)}$, $B_{(1-199)}$, $B_{200-456}$, $B_{(F61->C)}$, $B_{(C132->A)}$, $B_{(C132->S)}$, $B_{(C135->S)}$, $B_{(C-175->S)}$, $B_{(C132->A)(C135->S)(C175->S)}$, or $B_{(C-132->S)(C135->S)(C175->S)}$, wherein the portion of BPI Protein in the variant is designated by the letter B, followed by an amino acid sequence numbering assignment corresponding to that shown in Figures 8A-1, 8A-2, 8B-1 and 8B-2 for human BPI protein, wherein the mature N-terminus is designated as residue 1.

8.  A method according to any preceding claim wherein the gram-negative bacterial cell is *E.coli.*

9.  A method according to any preceding claim wherein the Bactericidal/Permeability Increasing Protein is produced in an active, soluble, form.

10. A method of recovering non-glycosylated Bactericidal/Permeability Increasing Protein from a gram-negative bacterial cell transformed or transfected with a vector encoding Bactericidal/Permeability Increasing Protein having no leader peptide attached thereto when expressed, and cultured so as to produce non-glycosylated Bactericidal/Permeability Increasing Protein in the cell, **characterised in that** the cell has been cultured at a temperature below 37°C, which method comprises the steps of:

    a) lysing the cell under conditions preventing the formation of a complex between the Bactericidal/Permeability Increasing Protein and LPS so as to release the Bactericidal/Permeability Increasing Protein from the cell; and

    b) isolating the Bactericidal/Permeability Increasing Protein so released.

11. A method of recovering non-glycosylated Bactericidal/Permeability Increasing Protein from a gram-negative bacterial cell transformed or transfected with a vector encoding Bactericidal/Permeability Increasing Protein having no leader peptide attached thereto when expressed, and cultured so as to produce non-glycosylated Bactericidal/Permeability Increasing Protein in the cell, **characterised in that** the cell has been cultured at a temperature below 37°C, which method comprises the steps of:

    a) lysing the cell under conditions permitting the formation of a complex between the Bactericidal/Permeability Increasing Protein and LPS;

b) isolating the resulting Bactericidal/Permeability Increasing Protein -LPS complex;

c) dissociating the Bactericidal/Permeability Increasing Protein -LPS complex so isolated, so as to release the Bactericidal/Permeability Increasing Protein from the Bactericidal/Permeability Increasing Protein -LPS complex; and

d) isolating the Bactericidal/Permeability Increasing Protein so released.

12. A method according to anyone of Claims 4, 5, 10 or 11, wherein the step of lysing the cell comprises removing the outer membrane of the cell and subsequently breaking open the resulting cell.

13. A method according to Claim 5 or 11, wherein the step of dissociating the Bactericidal/Permeability Increasing Protein-LPS complex comprises contacting the complex with divalent cations.

14. A method according to Claim 13, wherein the complex is contacted with 0.05M - 1.0M $MgCl_2$.

15. A method according to Claim 14, wherein the complex is contacted with 0.5M $MgCl_2$.

**Patentansprüche**

1. Verfahren zur Herstellung eines nicht glycosilierten bakteriziden/die Permeabilität erhöhenden Proteines unter Verwendung einer Gram-negativen Bakterienzelle, wobei das Verfahren umfaßt:

Transformieren oder Transfizieren der Zelle mit einem Vektor, der fiir das bakterizide/die Permeabilität erhöhende Protein kodiert, wobei das bakterizide/die Permeabilität erhöhende Protein kein Leader-Peptid mit sich verbunden hat, wenn es exprimiert wird,

Kultivieren der Zelle, die so transformiert oder transfiziert ist, um das nicht glycosilierte bakterizide/die Permeabilität erhöhende Protein in der Zelle herzustellen und

Gewinnen des nicht glycosilierten bakteriziden/die Permeabilität erhöhenden Proteines aus der Zelle, das so produziert wurde,

**dadurch gekennzeichnet, daß**
die Zelle bei einer Temperatur von unterhalb 37°C kultiviert wird.

2. Verfahren nach Anspruch 1, bei dem die Zelle bei einer Temperatur zwischen 25 - 36°C kultiviert wird.

3. Verfahren gemäß Anspruch 2, bei dem die Zelle bei einer Temperatur von 26°C kultiviert wird.

4. Verfahren gemäß einem der voranstehenden Ansprüche, bei dem die Gewinnung umfaßt:

(a) Lysieren der Zelle unter Bedingungen, die die Bildung eines Komplexes zwischen dem bakteriziden/die Permeabilität erhöhenden Protein und LPS verhütet, um so das bakterizide/die Permeabilität erhöhende Protein aus der Zelle freizusetzen und
(b) Isolieren des so freigesetzten bakteriziden/die Permeabilität erhöhenden Proteins.

5. Verfahren gemäß einem der Ansprüche 1 bis 3, bei dem das Gewinnen umfaßt:

(a) Lysieren der Zelle unter Bedingungen, die die Bildung eines Komplexes zwischen dem bakteriziden/die Permeabilität erhöhenden Protein und LPS zulassen,
(b) Isolieren des sich ergebenden Komplexes bakterizides/die Permeabilität erhöhendes Protein - LPS,
(c) Dissoziieren des Komplexes bakterizides/die Permeabilität erhöhendes Protein - LPS, der so isoliert wurde, um das bakterizide/die Permeabilität erhöhende Protein aus dem Komplex bakterizides/die Permeabilität erhöhendes Protein - LPS freizusetzen und
(d) Isolieren des so freigesetzten bakteriziden/die Permeabilität erhöhenden Proteins.

6. Verfahren gemäß einem der voranstehenden Ansprüche, bei dem das bakterizide/die Permeabilität erhöhende Protein das native menschliche Protein von 55 kDa ist, das die Aminosäuresequenz aufweist, die für das menschliche BPI-Protein in den Figuren 8A-1 und 8A-2 gezeigt ist und durch die cDNA-Sequenz kodiert wird, die in den Figuren 8B-1 und 8B-2 gezeigt ist oder eine BPI-Variante, die einen Abschnitt des BPI-Proteines umfaßt, wobei die Variante in der Lage ist, (a) an LPS zu binden, (b) mit dem BPI-Protein oder LBP um die Bindung an LPS in Wettbewerb zu treten und (c) die LPS-vermittelte Produktion von TNFα durch menschliche Monozyten zu inhibieren.

7. Verfahren gemäß Anspruch 6, bei dem die BPI-Variante $B_{(S351->A)}$, $B_{(D200->DP)}$, $B_{(1-199)}$, $B_{200-456}$, $B_{(F61->C)}$, $B_{(C132->A)}$, $B_{(C132->S)}$, $B_{(C135->S)}$, $B_{(C-175->S)}$, $B_{(C132->A)(C135->S)(C175->S)}$ oder $B_{(C-132->S)(C135->S)(C175->S)}$ ist, wobei der Abschnitt des BPI-Proteines in der Variante durch den Buchstaben B bezeichnet ist, gefolgt von einer Zuweisung der Numerierung der Aminosäuresequenz, die derjenigen entspricht, die in den Figuren 8A-1, 8A-2, 8B-1 und 8B-2 für das menschliche BPI-Protein gezeigt ist, bei dem der reife N-Terminus als Rest 1 bezeichnet ist.

8. Verfahren gemäß einem der voranstehenden Ansprüche, bei dem die Gram-negative bakterielle Zelle *E.coli* ist.

9. Verfahren gemäß einem der voranstehenden Ansprüche, bei dem das bakterizide/die Permeabilität erhöhende Protein in aktiver löslicher Form produziert wird.

10. Verfahren zum Gewinnen nicht glycosilierten bakteriziden/die Permeabilität erhöhenden Proteines aus einer Gram-negativen bakteriellen Zelle, die mit einem Vector transformiert oder transfiziert ist, der für bakterizides/die Permeabilität erhöhendes Protein kodiert, das kein Leader-Peptid an sich gebunden aufweist, wenn es exprimiert wird und so kultiviert wird, um nicht glycosiliertes bakterizides/die Permeabilität erhöhendes Protein in der Zelle zu produzieren, **dadurch gekennzeichnet, daß** die Zelle bei einer Temperatur von unterhalb 37°C kultiviert worden ist, welches Verfahren die Schritte umfaßt von:

   (a) Lysieren der Zelle unter Bedingungen, die die Bildung eines Komplexes zwischen dem bakteriziden/die Permeabilität erhöhenden Protein und LPS verhüten, um so das bakterizide/die Permeabilität erhöhende Protein aus der Zelle freizusetzen und
   (b) Isolieren des bakteriziden/die Permeabilität erhöhenden Proteins, das so freigesetzt ist.

11. Verfahren zum Gewinnen nicht glycosilierten bakteriziden/die Permeabilität erhöhenden Proteines aus einer Gram-negativen bakteriellen Zelle, die mit einem Vector transformiert oder transfiziert ist, der für ein bakterizides /die Permeabilität erhöhendes Protein kodiert, das kein Leader-Peptid an sich gebunden aufweist, wenn es exprimiert wird und so kultiviert wird, um nicht glycosiliertes bakterizides/die Permeabilität erhöhendes Protein in der Zelle zu produzieren, **dadurch gekennzeichnet, daß** die Zelle bei einer Temperatur von unterhalb 37°C kultiviert worden ist, welches Verfahren die Schritte umfaßt von:

   (a) Lysieren der Zelle unter Bedingungen, die die Bildung eines Komplexes zwischen dem bakteriziden/die Permeabilität erhöhenden Protein und LPS zulassen,
   (b) Isolieren des sich ergebenden Komplexes bakterizides/die Permeabilität erhöhendes Protein - LPS,
   (c) Dissoziieren des Komplexes bakterizides/die Permeabilität erhöhendes Protein - LPS, der so isoliert wurde, um das bakterizide/die Permeabilität erhöhende Protein aus dem Komplex bakterizides/die Permeabilität erhöhendes Protein - LPS freizusetzen und
   (d) Isolieren des so freigesetzten bakteriziden/die Permeabilität erhöhenden Proteins.

12. Verfahren gemäß Anspruch 10 oder 11, bei dem der Schritt des Lysierens der Zelle das Entfernen der äußeren Membran der Zelle und nachfolgendes Aufbrechen der erhaltenen Zelle umfaßt.

13. Verfahren gemäß Anspruch 11, bei dem der Schritt des Dissoziierens des Komplexes bakterizides/die Permeabilität erhöhendes Protein - LPS das Kontaktieren des Komplexes mit divalenten Kationen umfaßt.

14. Verfahren gemäß Anspruch 13, bei dem der Komplex mit 0,05 M - 1,0 M $MgCl_2$ in Kontakt gebracht wird.

15. Verfahren gemäß Anspruch 14, bei dem der Komplex mit 0,5 M $MgCl_2$ in Kontakt gebracht wird.

**Revendications**

1. Méthode de production de protéine non-glycosylée bactéricide/augmentant la perméabilité utilisant une cellule bactérienne gram-négative, la méthode comprenant:

   la transformation ou la transfection de la cellule avec un vecteur codant une protéine bactéricide/ augmentant la perméabilité, dans laquelle la protéine bactéricide/ augmentant la perméabilité ne possède pas de peptide leader attaché à elle lorsqu'elle est exprimée;
   la culture de la cellule ainsi transformée ou transfectée de manière à produire une protéine non glycosylée, bactéricide/augmentant la perméabilité dans la cellule; et
   la récupération à partir de la cellule de la protéine non glycosylée bactéricide/augmentant la perméabilité ainsi produite;

   **caractérisée en ce que** la cellule est cultivée à une température inférieure à 37°C.

2. Méthode selon la revendication 1, dans laquelle la cellule est cultivée à une température comprise entre 25 et 36°C.

3. Méthode selon la tevendication 2, dans laquelle la cellule est cultivée à une température de 26°C.

4. Méthode selon l'une quelconque des revendications précédentes dans laquelle la récupération comprend:

   (a) la lyse de la cellule dans les conditions empêchant la formation d'un complexe entre la protéine bactéricide/ augmentant la perméabilité et LPS de manière à libérer la protéine bactéricide/augmentant la perméabilité à partir de la cellule; et
   (b) l'isolement de la protéine bactéricide/augmentant la perméabilité ainsi libérée.

5. Méthode selon l'une quelconque des revendications 1 à 3 dans laquelle la récupération comprend:

   (a) la lyse de la cellule dans des conditions permettant la formation d'un complexe entre la protéine bactéricide/ augmentant la perméabilité et LPS;
   (b) l'isolement du complexe protéine bactéricide/augmentant la perméabilité-LPS;
   (c) la dissociation du complexe protéine bactéricide/augmentant la perméabilité-LPS, de manière à libérer la protéine bactéricide/augmentant la perméabilité du complexe protéine bactéricide/augmentant la perméabilité-LPS; et
   (d) l'isolement de la protéine bactéricide/augmentant la perméabilité ainsi libérée.

6. Méthode selon l'une quelconque des revendications précédentes dans laquelle la protéine bactéricide/augmentant la perméabilité est la protéine humaine 55 kD native ayant la séquence d'acides aminés montrée pour la protéine humaine BPI sur les figures 8A-1 et 8A-2, et codée par la séquence d'ADNc présentée sur les figures 8B-1 et 8B-2, ou un variant BPI qui comprend une partie d'une protéine BPI, lequel variant est capable (a) de se lier à LPS, (b) d'être un compétiteur de la protéine BPI ou LBP pour la liaison à LPS, et (c) d'inhiber la production de TNFx à médiation LPS par les monocytes humains.

7. Méthode selon la revendication 6, dans laquelle le variant BPI est ($B_{(S351 \rightarrow A)}$, $B_{(D200 \rightarrow DP)}$, $B_{(1-199)}$, $B_{200-456}$, $B_{(F61 \rightarrow C)}$, $B_{(C132 \rightarrow A)}$, $B_{(C132 \rightarrow S)}$, $B_{(C135 \rightarrow S)}$, $B_{(C175 \rightarrow S)}$ $B_{(C132 \rightarrow A)(C135 \rightarrow S)(C175 \rightarrow S)}$ ou $B_{(C-132 \rightarrow S)(C135 \rightarrow S)(C175 \rightarrow S)}$, dans lequel la partie de la protéine BPI dans le variant est désignée par la lettre B, suivie d'une affectation de numérotation de séquence d'acide aminé correspondant à celle représentée sur les figures 8A-1, 8A-2, 8B-1 et 8B-2 pour la protéine BPI humaine, dans laquelle l'extrémité N-terminale mature est désignée comme résidu 1.

8. Méthode selon l'une quelconque des revendications précédentes dans laquelle la bactérie gram-négative est *E. coli.*

9. Méthode selon l'une quelconque des revendications précédentes dans laquelle la protéine bactéricide/augmentant la perméabilité est produite sous une forme active, soluble.

10. Méthode de récupération de protéine bactéricide/augmentant la perméabilité à partir d'une cellule bactérienne gram-négative transformée ou transfectée avec un vecteur codant la protéine bactéricide/augmentant la perméabilité sans peptide leader attaché à celle-ci lorsqu'elle est exprimée et cultivée de manière à produire dans la cellule la protéine non glycosylée bactéricide/augmentant la perméabilité, **caractérisée en ce que** la cellule a été cultivée à une température inférieure à 37°C, laquelle méthode comprend les étapes de:

a) lyse de la cellule dans des conditions empêchant la formation d'un complexe entre la protéine bactéricide/augmentant la perméabilité et LPS de manière à libérer à partir de la cellule la protéine bactéricide/augmentant la perméabilité; et
b) isolement de la protéine bactéricide/augmentant la perméabilité ainsi libérée.

11. Méthode de récupération de protéine non glycosylée bactéricide/augmentant la perméabilité à partir d'une cellule bactérienne gram-négative transformée ou transfectée avec un vecteur codant la protéine bactéricide/augmentant la perméabilité sans peptide leader attaché à celle-ci lorsqu'elle est exprimée, et cultivée de manière à produire dans la cellule la protéine non glycosylée bactéricide/sugmentant la perméabilité, **caractérisée en ce que** la cellule a été cultivée à une tempétature inférieure à 37°C, laquelle méthode comprend les étapes de:

a) lyse de la cellule dans des conditions permettant la formation d'un complexe entre la protéine bactéricide/augmentant la perméabilité et LPS;
b) isolement du complexe protéine bactéricide/augmentant la perméabilité-LPS;
c) dissociation du complexe protéine bactéricide/augmentant la perméabilité-LPS ainsi isolé de manière à libérer la protéine bactéricide/augmentant la perméabilité à partir du complexe protéine bactéricide/augmentant la perméabilité-LPS; et
d) isolement de la protéine bactéricide/augmentant la perméabilité ainsi libérée.

12. Méthode selon l'une quelconque des revendications 4, 5, 10 ou 11, dans laquelle l'étape de lyse de la cellule comprend l'élimination de la membrane externe de la cellule est ultérieurement la rupture de la cellule obtenue.

13. Méthode selon la revendication 5 ou 11, dans laquelle l'étape de dissociation du complexe protéine bactéricide/augmentant la perméabilité-LPS comprend la mise en contact du complexe avec des cations divalents.

14. Méthode selon la revendication 13, dans laquelle le complexe est mis en contact avec $MgCl_2$ 0,05M-1,0M.

15. Méthode selon la revendication 14, dans laquelle le complexe est mis en contact avec $MgCl_2$ 0,5M.

FIG. I

## BPI MUTAGENIC PRIMERS

### C-TERMINAL TRUNCATION OF BPI

### 25KD Pro 212 TGA

```
AA.  205                          210      212
     Ile  Asn  Tyr  Gly  Leu  Val  Ala  Pro  Ter.Bam  HI

     TAG  AAC  TAT  GGT  CTG  GTG  GCA  CCT  TGA   GGATCCGCG

COMP
         3'   ATA  CCA  GAC  CAC  CGT  GGA  ACT   CCTAGGCGC 5'

OLIGO   459:

    5'  CGCGGATCC        TCA  AGG  TGC  CAC  CAG  ACC  ATA  3'
```

# FIG. 2

## BPI MUTAGENIC PRIMERS

### C-TERMINAL TRUNCATION OF BPI

### 38KD Pro 337 TGA

```
AA.  330                          335      337
     Pro  Thr  Gly  Leu  Thr  Phe  Tyr  Pro Ter  Bam  HI
     CCC  ACC  GGC  CTT  ACC  TTC  TAC  CCT  TGA  GGATCCGCG

COMP
         3'CCG       GAA  TGG  AAG  ATG  GGA  ACT  CCTAGGCGC 5'

OLIGO   460:

    5'   CGCGGATCC       TCA  AGG  GTA  GAA  GGT  AAG  GCC 3'
```

# FIG. 3

**BPI MUTAGENIC PRIMERS**

**C-TERMINAL TRUNCATION OF BPI**

**PREFERED ATG 5'HIND III:**

```
AA.         -31                    -26
      HINd III Start   Met Arg  Glu  Asn  Met  Arg
      CCCAAGCTT  GCC   ACC  ATG  AGA  GAG  AAC  ATG  GCC

OLIGO 458:
5'CCCAAGCTT  GCC  ACC  ATG  AGA  GAG  AAC  ATG  GCC  3'
```

# FIG.4

FIG. 5

FIG. 6A

# FIG. 6B

```
                    |
                    |
                 BamHI
                  4.00
```

```
 -16        -8              +171   +176
   |         |                |      |
     AAAAAAACC CGAGATCCGCGGATC CTTTCCT
     ------------------------------
        AvaIXho2Sst2 BamHI
```

No sites for:  SmaI, PstI, BglII, XbaI, SstI


The nucleotide sequence presented above was
determined by V.A. Luckow.

FIG. 7

FIG. 8A-1

-30

-20

-10

-20

Mouse LBP
Rabbit LBP
Human LBP
Human BPI
Bovine BPI

10

20

Mouse LBP
Rabbit LBP
Human LBP
Human BPI
Bovine BPI

FIG.8A-2

|  |  |  |  | 30 |  |  |  |  |  |  |  |  |  |  | 40 |  |  |  |  |  |  |  |  | 50 |
|--|--|--|--|--|--|--|--|--|--|--|--|--|--|--|--|--|--|--|--|--|--|--|--|--|

```
                  30                              40                        50
Q  R  E  L  Y  R  I  T  L  P  D  F  S  G  D  F  K  I  K  A  V  G  R  G  Q
Q  R  K  L  L  E  V  T  L  P  D  S  D  G  D  F  R  I  K  H  F  G  R  A  Q
Q  S  E  L  L  R  I  T  L  P  D  F  T  G  D  L  R  I  P  H  V  G  R  G  R
Q  K  E  L  K  R  I  K  I  P  D  Y  S  D  S  F  K  I  K  H  L  G  K  G  H
Q  K  E  L  E  K  I  T  I  P  N  F  S  G  N  F  K  I  K  Y  L  G  K  G  Q

                          60                          70
Mouse   LBP   Y  E  F  H  S  L  E  I  Q  N  C  E  L  R  G  S
Rabbit  LBP   Y  K  F  Y  S  L  K  I  P  R  F  E  L  L  R  G  T  L  R  P  L  P  G  Q  G
Human   LBP   Y  E  F  H  S  L  N  I  H  S  C  E  L  L  H  S  A  L  R  P  V  P  G  Q  G
Human   BPI   Y  S  F  Y  S  M  D  I  R  E  F  Q  L  P  S  S  Q  I  S  M  V  P  N  V  G
Bovine  BPI   Y  S  F  F  S  M  V  I  Q  G  F  N  L  P  N  S  Q  I  R  P  L  P  D  K  G

              80                          90                        100
L  S  L  D  I  S  D  A  Y  I  H  V  R  G  S  W  K  V  R  K  A  F  L  R  L
L  S  L  S  I  S  D  S  S  I  R  V  Q  G  R  W  K  V  R  K  S  F  F  K  L
L  K  F  S  I  S  N  A  N  I  K  I  S  G  K  W  K  A  Q  K  R  F  L  K  M
L  D  L  S  I  R  D  A  S  I  K  I  R  G  K  W  K  A  R  K  N  F  I  K  L
```

EP 0 726 944 B1

FIG. 8A-3

```
                                              110                                    120
              !   *   *        !  !  *           *  !  !           *      *  *  !  !        !   *
Rabbit  LBP   K  N  S  F  D  L  Y  V  K  G  L  T  I  S  V  H  L  V  L  G  S  E  -  S  S
Human   LBP   Q  G  S  F  D  V  S  V  K  G  I  S  I  S  V  N  L  L  L  G  S  E  -  S  S
Human   BPI   S  G  N  F  D  L  S  I  E  G  M  S  I  S  A  D  L  K  L  G  S  N  P  T  S
Bovine  BPI   G  G  N  F  D  L  S  V  E  G  I  S  I  L  A  G  L  N  L  G  Y  D  P  A  S
              !  !  *  *  !  !        !  *        !  *        !        *        *  *              !           *
                                 130                            140                                    150
              *  !  !  *  !  *     *  *  *  *  *  !  *        *  !        !        !        !
              G  R  P  T  V  T  T  S  S  C  S  S  D  I  Q  N  V  E  L  D  I  E  G  -  D
              G  R  P  T  V  T  A  S  S  C  S  S  D  I  A  D  V  E  V  D  M  S  G  -  D
              G  K  P  T  I  T  C  S  S  C  S  S  H  I  N  S  V  H  V  H  I  S  K  S  K
              G  H  S  T  V  T  C  S  S  C  S  S  G  I  N  T  V  R  I  M  I  S  G  S  S
              *        *        *  !  *  *  *  *  *        *  !        *                 !  !        !
                                         160                                  170
                    *  !  !  *        *              !  *                          !  !     !  !   .     !  *
Rabbit  LBP   L  E  E  L  L  N  L  L  Q  S  Q  I  D  A  R  L  R  E  V  L  E  S  K  I  C
Human   LBP   L  G  W  L  L  N  L  F  H  N  Q  I  E  S  K  F  Q  K  V  L  E  S  R  I  C
Human   BPI   V  G  W  L  I  Q  L  F  H  K  K  I  E  S  A  L  R  N  K  M  N  S  Q  V  C
Bovine  BPI   L  G  W  L  I  Q  L  F  R  K  R  I  E  S  L  L  Q  K  S  M  T  R  K  I  C
              !  !  *  !  !  *  !        !        *  !  !        !              !                    *
                                    180                            190                         25k/ 30k
                       !        *        *  *  *  *  *  *  *  *  *  *  !  *        !     *
              R  Q  I  E  E  A  V  T  A  H  L  Q  P  Y  L  Q  T  L  P  V  T  T  Q  I  D
              E  M  I  Q  K  S  V  S  S  D  L  Q  P  Y  L  Q  T  L  P  V  T  T  E  I  D
              E  K  V  T  N  S  V  S  S  K  L  Q  P  Y  F  Q  T  L  P  V  M  T  K  I  D
              E  V  V  T  S  T  V  S  S  K  L  Q  P  Y  F  Q  T  L  P  V  T  T  K  L  D
              !        !  !           *  . !  !  !  *  *  *  *  !  *  *  *  *        *  !           *
```

EP 0 726 944 B1

## FIG.8A-4

```
                                     210                              220
      !  !  *     !  !  *  !  *     !  !  *  !  !  *  *     !  *     !  !  !  *
Rabbit  LBP   S  F  A  G  I  D  Y  S  L  M  E  A  P  R  A  T  A  G  M  L  D  V  M  F  K
Human   LBP   S  F  A  D  I  D  Y  S  L  V  E  A  P  R  A  T  A  Q  M  L  E  V  M  F  K
Human   BPI   S  V  A  G  I  N  Y  G  L  V  A  P  P  A  T  T  A  E  T  L  D  V  Q  M  K
Bovine  BPI   K  V  A  G  V  D  Y  S  L  V  A  P  P  R  A  T  A  N  N  L  D  W  L  L  K
      !  *  !        *     *  !  !  !  *        *  *        *  !              *

                       230                     240                           250
      *  *  !  !        *  !  !  *  !        *  !        !        !  !  !        *
      G  E  I  F  P  L  D  H  R  S  P  V  D  F  L  A  P  A  M  N  L  P  E  A  H
      G  E  I  F  H  R  N  H  R  S  P  V  T  L  L  A  A  V  M  S  L  P  E  E  H
      G  E  F  Y  S  E  N  H  H  N  P  P  P  F  A  P  P  V  M  E  F  P  A  A  H
      G  E  F  F  S  L  A  H  R  S  P  P  P  F  A  P  P  A  L  A  F  A  S  D  H
      *  *  !  .  !        *        *  !  !  !  !  !  !              !        *

                          260                        270
         *  *  *  !     !  *  *  *  !  *  *  *  *  !  !  !  *  !           *  !
Rabbit  LBP   S  R  M  V  Y  F  S  I  S  D  Y  V  F  N  T  A  S  L  A  Y  H  K  S  G  Y
Human   LBP   N  K  M  V  Y  F  A  I  S  D  Y  V  F  N  T  A  S  L  V  Y  H  E  E  G  Y
Human   BPI   D  R  M  V  Y  L  G  L  S  D  Y  F  F  N  T  A  G  L  V  Y  Q  E  A  G  V
Bovine  BPI   D  R  M  V  Y  L  G  I  S  D  Y  F  F  N  T  A  G  F  V  Y  Q  K  A  G  A
      !  !  *  *  *  !  !     *  *  *  !  *  *  *  *  !     !  *  !        !  *

                          280                     290                        300
         !  !  !  !  !  *     *     *     !     !  !  *     *  *  *  !  *  !  !
      W  N  F  S  I  T  D  A  M  V  P  A  D  L  N  I  R  R  T  T  K  S  F  R  P
      L  N  F  S  I  T  D  D  M  I  P  P  D  S  N  I  R  L  T  T  K  S  F  R  P
      L  K  M  T  L  R  D  D  M  I  P  K  E  S  K  F  R  L  T  T  K  F  F  G  T
      L  N  L  T  L  R  D  D  M  I  P  K  E  S  K  F  R  L  T  T  K  F  F  G  I
      !           !  !  !  *  !  *  !  *  !  !  !  !  !  !  *  !  *  *  *  !  *  !
```

FIG.8A-5

<table>
<tr><td></td><td></td><td>310</td><td></td><td></td><td></td><td></td><td>320</td><td></td></tr>
</table>

Rabbit  LBP   F V P L L A N L Y P N M N L E L Q G T V N S E Q L
Human   LBP   F V P R L A R L Y P N M N L E L Q G S V P S A P L L
Human   BPI   F L P E V A K K F P N M K I Q I H V S A S T P P H
Bovine  BPI   L I P Q V A K M F P P M Q M Q L F I W A S L P P K

330                                     340                         350

V N L S T E N L L E E P E M D I E A L V V L P S S
L N F S P G N L S V D P Y M E I D A F V L L P S S
L S V Q P T G L T F Y P A V D V Q A F A V L P N S
L T M K P S S L D L I F V L D T Q A F A I L P N S

360                                     370

Rabbit  LBP   A R E P V F R L G V A T N V S A T L T L N T R K I
Human   LBP   S K E P V F R L S V A T N V S A T L T F N T S K I
Human   BPI   S L A S L F L I G M H T T G S M E V S A E S N R L
Bovine  BPI   S L D P L F L L E M N L N L S V V V G A K S D R L

380                                     390                         400

T G F L K P G R L Q V E L K E S K V G G F N V E L
T G F L K P G K V K V E L K E S K V G L F N A E L
V G E L K L D R L L L E L K H S N I G P F P V E L
I G E L R L D K L L L E L K H S D I G P F S V E S

EP 0 726 944 B1

310                                         320

! ! * ! * ! ! * ! * ! ! ! ! ! ! ! !

Rabbit  LBP     F V P L L A N L Y P N M N L E L Q G T V N S E Q L
Human   LBP     F V P R L A R L Y P N M N L E L Q G S V P S A P L
Human   BPI     F L P E V A K K F P N M K I Q I H V S A S T P P H
Bovine  BPI     L I P Q V A K M F P P M Q M Q L F I W A S L P P K

      * ! * ! ! * * ! ! ! ! !

330                                         340                                     350

! ! ! ! * ! ! ! * ! * * ! *

V N L S T E N L L E E P E M D I E A L V V L P S S
L N F S P G N L S V D P Y M E I D A F V L L P S S
L S V Q P T G L T F Y P A V D V Q A F A V L P N S
L T M K P S S L D L I F V L D T Q A F A I L P N S

! ! * * ! ! * ! ! * * ! *

360                                         370

! ! ! * ! ! ! ! ! ! ! * ! ! ! ! ! ! ! !

Rabbit  LBP     A R E P V F R L G V A T N V S A T L T L N T R K I
Human   LBP     S K E P V F R L S V A T N V S A T L T F N T S K I
Human   BPI     S L A S L F L I G M H T T G S M E V S A E S N R L
Bovine  BPI     S L D P L F L L E M N L N L S V V V G A K S D R L

! ! ! * ! ! ! ! ! ! !

380                                         390                                     400

! * ! * ! ! ! ! * * * ! * ! ! * * ! * !

T G F L K P G R L Q V E L K E S K V G G F N V E L
T G F L K P G K V K V E L K E S K V G L F N A E L
V G E L K L D R L L L E L K H S N I G P F P V E L
I G E L R L D K L L L E L K H S D I G P F S V E S

* ! * ! ! ! ! ! * * * ! * ! * ! ! ! *

```
                                    410                               420
          * ! ! ! ! * * ! ! ! !        ! * !        *        * * !              *
Rabbit LBP L E A L L N Y Y I L N N L Y P K V N E K L A H R F
Human LBP  L E A L L N Y Y I L N T F Y P K F N D K L A E G F
Human BPI  L Q D I M N Y I V P I L V L P R V N E K L Q K G F
Bovine BPI L Q S V I N Y V M P T I V L P V I N K K L Q K G F
          * !        * *        !        ! ! *        *        * * ! ! ! *

             430                               440                      450
          * * * ! !        ! * ! ! !    * *        !        * *        * !
          P L P L L R H I Q L Y D L L L Q T H E N F L L V G
          P L P L L K R V Q L Y D L G L Q I H K D F L F L G
          P L P T P A R V Q L Y N V V L Q P H Q N F L L F G
          P L P L P A Y I E L F N L T L Q P Y Q D F L L F G
          * * *        ! !        *        * * !        !        * * ! ! *


          * !        ! *        ! !
Rabbit LBP A N I Q Y R R V
Human LBP  A N V Q Y M R V
Human BPI  A D V V Y K
Bovine BPI A D V Q Y S D
          * ! *        *
```

EP 0 726 944 B1

```
BPI cDNA
  1 CAG GCC TTG AGG TTT TGG CAG CTC TGG AGG ATG AGA GAC AAC ATG GCC   48
  1                                             Met Arg Glu Asn Met Ala   6

 49 AGG GGC CCT TGC AAC GCG CCG AGA TGG GTG TCC CTG ATG GTG CTC GTC   96
  7 Arg Gly Pro Cys Asn Ala Pro Arg Trp Val Ser Leu Met Val Leu Val   22

 97 GCC ATA GGC ACC GCC GTG ACA GCG GCC GTC AAC CCT GGC GTC GTG GTC  144
 23 Ala Ile Gly Thr Ala Val Thr Ala Ala Val Asn Pro Gly Val Val Val   38

145 AGG ATC TCC CAG AAG GGC CTG GAC TAC GCC AGC CAG CAG GGG ACG GCC  192
 39 Arg Ile Ser Gln Lys Gly Leu Asp Tyr Ala ser Gln Gln Gly Thr Ala   54

193 GCT CTG CAG AAG GAG CTG AAG AGG ATC AAG ATT CCT GAC TAC TCA GAC  240
 55 Ala Leu Gln Lys Glu Leu Lys Arg Ile Lys Ile Pro Asp Tyr Ser Asp   70

241 AGC TTT AAG ATC AAG CAT CTT GGG AAG GGG CAT TAT AGC TTC TAC AGC  288
 71 Ser Phe Lys Ile Lys His Leu Gly Lys Gly His Tyr Ser Phe Tyr Ser   86

289 ATG GAC ATC CGT GAA TTC CAG CTT CCC AGT TCC CAG ATA AGC ATG GTG  336
 87 Met Asp Ile Arg Glu Phe Gln Leu Pro Ser Ser Gln Ile Ser Met Val  102

337 CCC AAT GTG GGC CTT AAG TTC TCC ATC AGC AAC GCC AAT ATC AAG ATC  384
103 Pro Asn Val Gly Leu Lys Phe Ser Ile Ser Asn Ala Asn Ile Lys Ile  118

385 AGC GGG AAA TGG AAG GCA CAA AAG AGA TTC TTA AAA ATG AGC GGC AAT  432
119 Ser Gly Lys Trp Lys Ala Gln Lys Arg Phe Leu Lys Met Ser Gly Asn  134

433 TTT GAC CTG AGC ATA GAA GGC ATG TCC ATT TCG GCT GAT CTG AAG CTG  480
135 Phe Asp Leu Ser Ile Glu Gly Met Ser Ile Ser Ala Asp Leu Lys Leu  150

481 GGC AGT AAC CCC ACG TCA GGC AAG CCC ACC ATC ACC TGC TCC AGC TGC  528
151 Gly Ser Asn Pro Thr Ser Gly Lys Pro Thr Ile Thr Cys Ser Ser Cys  166

529 AGC AGC CAC ATC AAC AGT GTC CAC GTG CAC ATC TCA AAG AGC AAA GTC  576
167 Ser Ser His Ile Asn Ser Val His Val His Ile Ser Lys Ser Lys Val  182

577 GGG TGG CTG ATC CAA CTC TTC CAC AAA AAA ATT GAG TCT GCG CTT CGA  624
183 Gly Trp Leu Ile Gln Leu Phe His Lys Lys Ile Glu Ser Ala Leu Arg  198

625 AAC AAG ATG AAC AGC CAG GTC TGC GAG AAA GTG ACC AAT TCT GTA TCC  672
199 Asn Lys Met Asn Ser Gln Val Cys Glu Lys Val Thr Asn Ser Val Ser  214

673 TCC AAG CTG CAA CCT TAT TTC CAG ACT CTG CCA GTA ATG ACC AAA ATA  720
215 Ser Lys Leu Gln Pro Tyr Phe Gln Thr Leu Pro Val Met Thr Lys Ile  230

721 GAT TCT GTG GCT GGA ATC AAC TAT GGT CTG GTG GCA CCT CCA GCA ACC  768
231 Asp Ser Val Ala Gly Ile Asn Tyr Gly Leu Val Ala Pro Pro Ala Thr  246
    ...
769 ACG GCT GAG ACC CTG GAT GTA CAG ATG AAG GGG GAG TTT TAC AGT GAG  816
247 Thr Ala Glu Thr Leu Asp Val Gln Met Lys Gly Glu Phe Tyr Ser Glu  262

817 AAC CAC CAC AAT CCA CCT CCC TTT GCT CCA CCA GTG ATG GAG TTT CCC  864
263 Asn His His Asn Pro Pro Pro Phe Ala Pro Pro Val Met Glu Phe Pro  278
```

## FIG.8B-I

EP 0 726 944 B1

```
865  GCT GCC CAT GAC CGC ATG GTA TAC CTG GGC CTC TCA GAC TAC TTC TTC  912
279  Ala Ala His Asp Arg Met Val Tyr Leu Gly Leu Ser Asp Tyr Phe Phe  294

913  AAC ACA GCC GGG CTT GTA TAC CAA GAG GCT GGG GTC TTG AAG ATG ACC  960
295  Asn Thr Ala Gly Leu Val Tyr Gln Glu Ala Gly Val Leu Lys Met Thr  310

961  CTT AGA GAT GAC ATG ATT CCA AAG GAG TCC AAA TTT CGA CTG ACA ACC  1008
311  Leu Arg Asp Asp Met Ile Pro Lys Glu Ser Lys Phe Arg Leu Thr Thr  326

1009 AAG TTC TTT GGA ACC TTC CTA CCT GAG GTG GCC AAG AAG TTT CCC AAC  1056
327  Lys Phe Phe Gly Thr Phe Leu Pro Glu Val Ala Lys Lys Phe Pro Asn  342

1057 ATG AAG ATA CAG ATC CAT GTC TCA GCC TCC ACC CCG CCA CAC CTG TCT  1104
343  Met Lys Ile Gln Ile His Val Ser Ala Ser Thr Pro Pro His Leu Ser  358

1105 GTG CAG CCC ACC GGC CTT ACC TTC TAC CCT GCC GTG GAT GTC CAG GCC  1152
359  Val Gln Pro Thr Gly Leu Thr Phe Tyr Pro Ala Val Asp Val Gln Ala  374

1153 CTT GCC GTC CTC CCC AAC TCC TCC CTG GCT TCC CTC TTC CTG ATT GGC  1200
375  Leu Ala Val Leu Pro Asn Ser Ser Leu Ala Ser Leu Phe Leu Ile Gly  390

1201 ATG CAC ACA ACT GGT TCC ATG GAG GTC AGC GCC GAG TCC AAC AGG CTT  1248
391  Met His Thr Thr Gly Ser Met Glu Val Ser Ala Glu Ser Asn Arg Leu  406

1249 GTT GGA GAG CTC AAG CTG GAT AGG CTG CTC CTG GAA CTG AAG CAC TCA  1296
407  Val Gly Glu Leu Lys Leu Asp Arg Leu Leu Leu Glu Leu Lys His Ser  422

1297 AAT ATT GGC CCC TTC CCG GTT GAA TTG CTG CAG GAT ATC ATG AAC TAC  1344
423  Asn Ile Gly Pro Phe Pro Val Glu Leu Leu Gln Asp Ile Met Asn Tyr  438

1345 ATT GTA CCC ATT CTT GTG CTG CCC AGG GTT AAC CAG AAA CTA CAG AAA  1392
439  Ile Val Pro Ile Leu Val Leu Pro Arg Val Asn Gln Lys Leu Gln Lys  454

1393 GGC TTC CCT CTC CCG ACG CCG GCC AGA GTC CAG CTC TAC AAC GTA GTG  1440
455  Gly Phe Pro Leu Pro Thr Pro Ala Arg Val Gln Leu Tyr Asn Val Val  470

1441 CTT CAG CCT CAC CAG AAC TTC CTG CTG TTC GGT GCA GAC GTT GTC TAT  1488
471  Leu Gln Pro His Gln Asn Phe Leu Leu Phe Gly Ala Asp Val Val Tyr  486

1489 AAA TGA AGG CAC CAG GGG TGC CGG GGG CTG TCA GCC GCA CCT GTT CCT  1536
487  Lys ***                                                          488

1537 GAT GGG CTG TGG GGC ACC GGC TGC CTT TCC CCA GGG AAT CCT CTC CAG  1584

1585 ATC TTA ACC AAG AGC CCC TTG CAA ACT TCT TCG ACT CAG ATT CAG AAA  1632

1633 TGA TCT AAA CAC GAG GAA ACA TTA TTC ATT GGA AAA GTG CAT GGT GTG  1680

1681 TAT TTT AGG GAT TAT GAG CTT CTT TCA AGG GCT AAG GCT GCA GAG ATA  1728

1729 TTT CCT CCA GGA ATC GTG TTT CAA TTG TAA CCA AGA AAT TTC CAT TTG  1776

1777 TGC TTC ATG AAA AAA AAC TTC TGG TTT TTT TCA TGT G                1813
```

## FIG.8B-2

```
-31   M R E N M A R G P C N A P R W V S L M V L V A I G T A V T A
 -1   A V N P G V V V R I S Q K G L D Y A S Q Q G T A A L Q K E L
 30   K R I K I P D Y S D S F K I K H L G K G H Y S F Y S M D I R
 60   E F Q L P S S Q I S M V P N V G L K F S I S N A N I K I S G
 90   K W K A Q K R F L K M S G N F D L S I E G M S I S A D L K L
120   G S N P T S G K P T I T C S S C S S H I N S V H V H I S K S
150   K V G W L I Q L F H K K I E S A L R N K M N S Q V C E K V T
180   N S V S S K L Q P Y F Q T L P V M T K I D S V A G I N Y G L
210   V A P P A T T A E T L D V Q M K G E F Y S E N H H N P P P F
240   A P P V M E F P A A H D R M V Y L G L S D Y F F N T A G L V
270   Y Q E A G V L K M T L R D D M I P K E S K F R L T T K F F G
300   T F L P E V A K K F P N M K I Q I H V S A S T P P H L S V Q
330   P T G L T F Y P
```

# FIG.9

```
-31   M R E N M A R G P C N A P R W V S L M V L V A I G T A V T A
 -1   A V N P G V V V R I S Q K G L D Y A S Q Q G T A A L Q K E L
 30   K R I K I P D Y S D S F K I K H L G K G H Y S F Y S M D I R
 60   E F Q L P S S Q I S M V P N V G L K F S I S N A N I K I S G
 90   K W K A Q K R F L K M S G N F D L S I E G M S I S A D L K L
120   G S N P T S G K P T I T C S S C S S H I N S V H V H I S K S
150   K V G W L I Q L F H K K I E S A L R N K M N S Q V C E K V T
180   N S V S S K L Q P Y F Q T L P V M T K I D S V A G I N Y G L
210   V A P
```

# FIG.10

FIG. 11

FIG. 12

FIG. 13